(19) Europäisches Patentamt / European Patent Office / Office européen des brevets

(11) EP 4 180 425 A1

(12) EUROPEAN PATENT APPLICATION

(43) Date of publication:
**17.05.2023 Bulletin 2023/20**

(21) Application number: **21207457.9**

(22) Date of filing: **10.11.2021**

(51) International Patent Classification (IPC):
***C07D 301/22** (2006.01)*

(52) Cooperative Patent Classification (CPC):
**C07D 301/22**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Fritz-Haber-Institut Der Max-Planck-Gesellschaft
14195 Berlin (DE)**

(72) Inventors:
- **Kube, Pierre
15370 Petershagen (DE)**
- **Trunschke, Annette
12489 Berlin (DE)**
- **Schlögl, Robert
10825 Berlin (DE)**

(74) Representative: **Hoffmann Eitle
Patent- und Rechtsanwälte PartmbB
Arabellastraße 30
81925 München (DE)**

# (54) METHOD FOR PRODUCING PROPYLENE OXIDE

(57) The present invention relates to a method for producing propylene oxide directly from propane. The method comprises a step of heating a reaction mixture comprising propane and oxygen in a reactor to form a product mixture comprising propylene oxide. It also comprises a step of isolating the propylene oxide from the product mixture. The invention also relates to methods wherein the isolated propylene oxide is further reacted.

Figure 1.

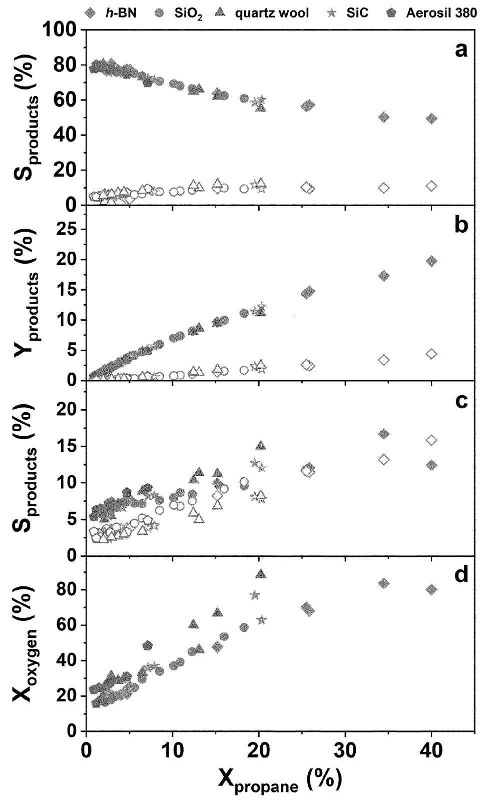


EP 4 180 425 A1

## Description

### Technical Field of the Invention

The present invention relates to a method for producing propylene oxide directly from propane. The method comprises a step of heating a reaction mixture comprising propane and oxygen in a reactor to form a product mixture comprising propylene oxide. It also comprises a step of isolating the propylene oxide from the product mixture.

### Background Art

The chemical industry, like all sectors of the economy, faces the challenge of bringing emissions of climate-damaging $CO_2$ to zero (Proc. Natl. Acad. Sci. U.S.A. 116, 11187-11194, (2019)). However, the synthesis of important intermediates, such as epoxides, is still associated with the release of large amounts of greenhouse gases. This is due to both the high energy input required for many of the process steps and insufficient selectivity of the underlying reactions, especially if they are oxidation reactions.

Propylene, as a key building block of the chemical industry, was produced globally at a scale of 130 million metric tonnes in 2019 (J. Energy Chem. 56, 193-202, (2021)). Propylene oxide is a major intermediate in the chemical industry for producing a large variety of consumer products including polyether polyols that are used in the manufacture of polyurethanes, propylene glycols as raw materials for the production of unsaturated polyester resins applied in the textile and construction industries, and propylene glycol ethers utilized as solvents in paints, inks, coatings and many other related applications (Baer et al Propylene Oxide (Wiley Online Library, 2012)).

Large-scale production of propylene oxide (globally approximately 10 million t/y in 2012) starts from propylene produced from crude oil fractions by steam cracking or fluid catalytic cracking (FCC). About 10% of all produced propylene is used for the manufacture of propylene oxide, using three main production technologies: the chlorohydrin process, coproduct routes, and liquid-phase epoxidation.

The traditional chlorohydrin process is performed in aqueous solution and requires two steps. The first step involves reaction of propylene with chlorine to deliver propylene chlorohydrin and the second is dehydrochlorination of the resultant propylene chlorohydrin in basic conditions to deliver propylene oxide. This delivers the propylene oxide in a mixture together with water and numerous by products: to isolate the propylene oxide, further condensation and distillation steps are required, and the water used as the solvent must be submitted to wastewater treatment.

The coproduct routes refer to the oxidation of propylene with organic hydroperoxides. These also require two steps. In a first step, organic hydroperoxides are generated by reaction of oxygen with alkenes at 120-150°C and elevated pressure (2-35 bar) in the absence of a catalyst. In the second step, epoxidation of propylene with the organic hydroperoxide is performed at 100-110°C and 35-40 bar pressure in an organic solvent applying a molybdenum salt as homogeneous catalyst. This process then requires extensive purification procedures to isolate the propylene oxide.

More recently liquid-phase epoxidation of propylene with hydrogen peroxide in methanol as solvent at 30-80°C and 10-30 bar has been commercialized. The catalyst is a titanium silicalite (TS-1). Hydrogen peroxide in the form of an aqueous solution is produced in a first step in the complex anthraquinone process: this is done on site to avoid the transport of large volumes of hydrogen peroxide solution. The product mixture contains, aside from propylene oxide, the solvent methanol, residual propylene, and byproducts for which reason dedicated separation and isolation technologies are required (Baer et al, Propylene Oxide (Wiley Online Library, 2012), and Nature 586, 708-713, (2020)).

Synthesis of propylene oxide from propylene using molecular oxygen has therefore been investigated (Catal. Rev. 57, 306-344, (2015)). Supported Ag catalysts modified by promoters and $TiO_2$-based systems have been used in heterogeneous oxidation of propylene by $O_2$.

One limitation of the processes above is that they start from propylene, which must itself be synthesised. This can be carried out by steam cracking of longer chain saturated hydrocarbons, fluid catalytic cracking (FCC) of crude oil fractions, or alternatively directly using propane in, for example, the known Oleflex process, which requires high temperature conditions and continuous regeneration of the catalyst to transform propane to propylene.

Meanwhile, the formation of propylene oxide has never been reported in studies of propane oxidation with molecular oxygen over either redox-active selective oxidation catalysts or solid materials such as nitrides and carbides. There is a report on the formation of propylene oxide in propane oxidation at temperatures between 400 und 430°C on $SiO_2$-supported $V_2O_5$ catalysts (J. Catal. 336, 23-32, (2016)). But in this case, $N_2O$ was used as oxidant, instead of molecular oxygen.

### Technical Problem

A demand therefore remains for the development of an improved method of producing sufficient amounts of propylene oxide for use on an industrial scale. The method should make more efficient use of time and resources: it

should be based on a simple transformation having a minimum number of steps. In this regard, it is desired that there is a minimum number of steps for making reactants from raw materials, and a minimum number of components in the reaction to avoid complexity. For similar reasons, it is also desired that the propylene oxide can be isolated in a minimum number of separation steps. Finally, there is also a demand for a method which reduces generation of greenhouse gases including carbon dioxide, and also avoids generation of contaminated solvents to reduce the environmental impact of the method.

**Summary of the Invention**

The present invention surprisingly solves the above problem by providing a method for producing propylene oxide, the method comprising: heating a reaction mixture comprising propane and oxygen in a reactor to form a product mixture comprising propylene oxide, and isolating the propylene oxide from the product mixture. It has surprisingly been found that this method delivers sufficient amounts of propylene oxide for use on an industrial scale. This is a clear advantage over e.g. the catalytic syntheses of propylene oxide from propylene using molecular oxygen described above, where the selectivity for propylene oxide was too low for an industrial application.

It also makes very efficient use of time and resources, as it is based on the direct transformation of propane to propylene oxide, and only requires a single step. This is a clear improvement over the numerous routes starting from propylene discussed above, all of which require multiple steps especially when the synthesis of propylene is taken into account.

The transformation is also more resource efficient as it uses fewer reactants than the various methods discussed above: no auxiliary chemicals, solvents, or catalysts are required. This also helps to simplify the transformation further, as there are fewer components which may have an impact on the outcome of the reaction. This has significant advantages for use on an industrial scale, where each additional component adds significant complexity to the conditions and plant required.

Efficiency is also improved by the method of the invention because it uses reactants which are directly available from natural sources: propane can be obtained directly from natural gas, and oxygen is present in the air. In contrast, the numerous methods described above rely heavily on auxiliary chemicals, solvents, and catalysts which ultimately increase the amount of resources required to deliver the propylene oxide.

The method of the invention also minimizes generation of greenhouse gases including carbon dioxide, and also avoids generation of contaminated solvents. Again, this is to be contrasted with the methods discussed above which either produce significant amounts of carbon dioxide as a side product, or at least have a larger carbon footprint due to the large number of steps and reagents required.

The invention encompasses the following embodiments:

1. A method for producing propylene oxide, the method comprising:

   (i) heating a reaction mixture comprising propane and oxygen in a reactor to form a product mixture comprising propylene oxide; and
   (ii) isolating the propylene oxide from the product mixture.

2. The method according to Embodiment 1, wherein the reaction mixture is heated to at least 470°C under 1 - 5 atmospheres, and preferably wherein the reaction mixture is heated to 470°C to 510°C under 1 - 3 atmospheres.

3. The method according to any of Embodiment 1 or 2, wherein the propane is present in the reaction mixture in an amount of 5 mol% to 80 mol%, preferably 20 mol% to 70 mol%, based upon 100 mol% of the reaction mixture.

4. The method according to any of Embodiments 1, 2, or 3, wherein a) the oxygen is present in the reaction mixture in an amount of 5 mol% to 25 mol%, preferably 12 mol% to 18 mol%, based upon 100 mol% of the reaction mixture; and/or b) the reaction mixture comprises an inert gas, wherein the inert gas is preferably present in the reaction mixture in an amount of 5 mol% to 90 mol%, preferably 20 mol% to 70 mol%, preferably 25 mol% to 65 mol%, based upon 100 mol% of the reaction mixture.

5. The method according to any of Embodiments 1, 2, 3, or 4, wherein the mole ratio of propane to oxygen in the reaction mixture is 10:1 to 1:2.

6. The method according to any of Embodiments 1, 2, 3, 4, or 5, wherein the proportion of the propane in the reaction mixture that is converted into the product mixture is 10 to 60%, preferably 25 to 45%.

7. The method according to any of Embodiments 1, 2, 3, 4, 5, or 6, wherein the selectivity for propylene oxide is above 3%, preferably above 5%.

8. The method according to any of Embodiments 1, 2, 3, 4, 5, 6, or 7, wherein the yield of propylene oxide is 3% or more.

9. The method according to any of Embodiments 1, 2, 3, 4, 5, 6, 7, or 8, wherein the yield of carbon dioxide is less than 4%.

10. The method according to any of Embodiments 1, 2, 3, 4, 5, 6, 7, 8, or 9, wherein the reactor contains a solid material and the reaction mixture is in contact with the solid material.

11. The method according to Embodiment 10, wherein the solid material comprises at least one of silicon carbide (SiC), boron nitride (BN), and silicon dioxide ($SiO_2$) wherein the $SiO_2$ optionally comprises or consists of crystalline silica, quartz wool or fumed silica.

12. The method according to any of Embodiments 1, 2, 3, 4, 5, 6, 7, 8, 9, 10, or 11, wherein a redox-active catalyst is not present in the reactor.

13. The method according to any of Embodiments 1, 2, 3, 4, 5, 6, 7, 8, 9, 10, 11, or 12, wherein the reactor is a single reactor, and/or
the reactor is a tubular fixed bed reactor.

14. The method according to any of Embodiments 1, 2, 3, 4, 5, 6, 7, 8, 9, 10, 11, 12, or 13, further comprising introducing the reaction mixture comprising propane and oxygen into the reactor before (i).

15. The method according to any of Embodiments 1, 2, 3, 4, 5, 6, 7, 8, 9, 10, 11, 12, 13, or 14 further comprising (iii), (iv), (v), (vi), (vii), or (viii) after (ii):

(iii) reacting the propylene oxide to produce one or more polyether polyols, and optionally further reacting the one or more polyether polyols to produce one or more polyurethanes; or
(iv) reacting the propylene oxide to produce one or more propylene glycols, and optionally further reacting the one or more propylene glycols to produce one or more unsaturated polyester resins; or
(v) reacting the propylene oxide to produce propylene glycol ethers, and optionally producing paints, inks, and/or coatings comprising the propylene glycol ethers; or
(vi) reacting the propylene oxide to produce isopropanolamine; or
(vii) reacting the propylene oxide to produce propylene carbonate; or
(viii) reacting the propylene oxide to produce allyl alcohol, acetone, and/or propanal.

Where the present description refers to "preferred" embodiments/features, combinations of these "preferred" embodiments/features shall also be deemed as disclosed as long as this combination of "preferred" embodiments/features is technically meaningful.
Hereinafter, the use of the term "comprising" should be understood as disclosing, as a more restricted embodiment, the term "consisting of" as well, as long as this is technically meaningful.
Where the application refers to a ratio of x:y e.g. 1:2, this can be read also as the corresponding number e.g. 1/2 = 0.5. Hence, a lower ratio of x:y would be one in which the y is relatively larger than x than in the first ratio, e.g. 1:3 is a lower x:y ratio than 1:2.

**Brief Description of the Figures**

**Figure 1.** Performance of propane oxidation shown as a function of propane conversion using various solid materials in the reactor of example 1; (a) selectivity and (b) yield of propylene (filled symbols) and propylene oxide (open symbols); (c) selectivity to ethylene (filled symbols) and CO (open symbols), and (d) conversion of $O_2$.

**Figures 2, 3, 4, 5,** and **6** show the same information as figure 1 individually for each of examples 1a to 1e.

**Figure 7.** Selectivity for (a) propylene and (b) propylene oxide measured for three different feed compositions over

$SiO_2$ and plotted against propane conversion in examples 2a - 2c; and selectivity for (c) propylene and (d) propylene oxide measured for three different feed compositions over $SiO_2$ and plotted against oxygen conversion in examples 2a - 2c.

**Figure 8.** Selectivity for (a) ethylene and (b) CO measured for three different feed compositions over $SiO_2$ and plotted against propane conversion in examples 2a - 2c; and selectivity for (c) ethylene and (d) CO measured for three different feed compositions over $SiO_2$ and plotted against oxygen conversion in examples 2a - 2c.

**Figure 9.** Selectivity for (a) $CO_2$, (b) acrolein, (c) acetaldehyde and (d) propionaldehyde measured for three different feed compositions over $SiO_2$ in examples 2a - 2c.

**Figure 10.** Ratio of (a) $r_{PO}/r_{propylene}$ and (b) $r_{ethylene}/r_{propylene}$ measured for $SiO_2$ as a function of W/F in examples 2a - 2c.

**Figure 11.** Yields of propylene oxide as a function of propane conversion in Examples 2a - 2c.

**Figure 12.** Sum of the yields of propylene and propylene oxide as a function of propane conversion in Examples 2a - 2c.

**Figure 13.** Performance of propane oxidation in examples 3a - 3c: (a) selectivity for and (b) yield of propylene (filled symbols) and propylene oxide (open symbols), (c) selectivity for ethylene (filled symbols) and CO (open symbols), and (d) conversion of $O_2$ shown as a function of propane conversion.

**Figure 14.** Propane conversion as a function of the material bed height in examples 4a and 4b.

**Figure 15.** Conversion of propane as a function of the total flow measured with $SiO_2$ and SiC in examples 5a and 5b.

**Figure 16.** Arrhenius plot for the determination of the apparent activation energy for propane conversion measured with $SiO_2$ and SiC as filling materials in examples 6a and 6b.

**Figure 17.** Temperature programmed reaction mass spectrometry results for reference example 1a.

**Figure 18.** Temperature programmed reaction mass spectrometry results for reference example 1b.

**Figure 19.** Temperature programmed reaction mass spectrometry results for reference example 1c.

**Figure 20.** Temperature programmed reaction mass spectrometry results for reference example 2.

**Figure 21.** Temperature programmed reaction mass spectrometry results for reference example 3.

**Figure 22.** Flowchart of the one-step process of direct oxidation of propane to propylene, propylene oxide, ethylene and CO in comparison with the current PO processes starting from propane as the same raw material (Baer et al Propylene Oxide (Wiley Online Library, 2012)).

HPPO stands for Hydrogen Peroxide to Propylene Oxide and MTBE is Methyl tert-butyl ether.

**Figure 23.** Aspen HYSYS flow-sheet for direct oxidation of propane to propylene oxide.

### Detailed Description of the Invention

The final product of the method of the invention is propylene oxide, which has the following chemical formula:

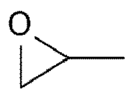

Propane is a starting material in the method of the invention, and has the chemical formula $CH_3CH_2CH_3$. Oxygen is a starting material in the method of the invention, and has the chemical formula $O_2$, also known as molecular

oxygen.

The atoms in the propane, oxygen, and propylene oxide in the invention may be present as any available isotopes. Therefore, the hydrogen atoms may be partially or completely replaced with deuterium or tritium, and one or more of the carbon atoms may be present as $^{12}C$, $^{13}C$, and/or $^{14}C$. Similarly, the oxygen atoms may be present as $^{16}O$, $^{17}O$, and $^{18}O$.

The method of the invention comprises (i) heating a reaction mixture comprising propane and oxygen in a reactor to form a product mixture comprising propylene oxide; and (ii) isolating the propylene oxide from the product mixture. (ii) is based on the product mixture formed in (i) and therefore takes place after (i). The method of the invention may also consist of (i) and (ii). The method is a single step reaction, meaning that a single direct chemical transformation of propane to propylene oxide occurs.

The reaction mixture is defined based on the contents of the reaction mixture in the reactor prior to the reaction to form the product mixture in (i). The reaction mixture comprises propane and oxygen. It does not include the solid material discussed later for the purposes of the amounts of components in the reaction mixture given in the following. The mol% values given are all based upon 100 mol% of the reaction mixture.

The propane may be obtained from natural gas processing or petroleum refining.

In the method of the invention, propane is preferably present in the reaction mixture in an amount of 5 mol% to 80 mol%, preferably 10 mol% to 75 mol%, preferably 20 mol% to 70 mol%, preferably 25 mol% to 65 mol%. Propane may also be present in the reaction mixture in an amount of 20 mol% to 40 mol%, preferably 25 mol% to 35 mol%, or 50 mol% to 70 mol%, preferably 55 mol% to 65 mol%. Amounts of propane in these ranges lead to an improved selectivity for propylene oxide relative to other products in the reaction, improving the efficiency of the method.

The oxygen may be present in the reaction mixture in an amount of 5 mol% to 25 mol%, 10 mol% to 20 mol%, preferably 12 mol% to 18 mol%. Higher amounts of oxygen lead to a higher conversion of propane in the reaction, thus improving the yield of propylene oxide and therefore the efficiency. The oxygen may be obtained from the liquification and distillation of air.

It is preferred that the source of oxygen in the reaction mixture is air. Air contains 21 mol% oxygen excluding water vapour. In this case, the reaction mixture comprises propane and air and may consist of propane and air. Using air in the reaction mixture improves the efficiency and simplicity of the reaction, as air can be obtained directly from the atmosphere and does not require a further liquification and distillation step. This further reduces the resources required by the inventive method. When air is present, the propane is preferably present in an amount of 5 mol% to 75 mol%, preferably 10 mol% to 75 mol%, preferably 20 mol% to 70 mol%, preferably 25 mol% to 65 mol%, and air is preferably present in the reaction mixture in an amount of 95 mol% to 25 mol%, 90 mol% to 25 mol%, preferably 80 mol% to 30 mol%, preferably 75 mol% to 35 mol%.

The reaction mixture may also comprise an inert gas. It may therefore consist of an inert gas, propane and oxygen. The inert gas does not undergo a chemical reaction during the formation of the product mixture from the reaction mixture in the invention. It may comprise a noble gas (i.e. helium, neon, argon, krypton, or xenon) and/or nitrogen. A single inert gas may be used or a mixture of one or more inert gases may be used together. It is preferred that the inert gas is helium and/or nitrogen, and it is most preferred that the inert gas is nitrogen as nitrogen is more readily available and further reduces the resources required by the inventive method. The inert gas is preferably present in the reaction mixture in an amount of 5 mol% to 90 mol%, preferably 10 mol% to 75 mol%, preferably 20 mol% to 70 mol%, preferably 25 mol% to 65 mol%. The inert gas may also be present in the reaction mixture in an amount of 20 mol% to 30 mol%, or 50 mol% to 60 mol%. Lower amounts of inert gas lead to higher selectivity for propylene oxide relative to other products in the reaction, improving the efficiency of the method.

When air is the source of oxygen in the reaction mixture, the inert gases are those naturally contained in air: nitrogen, argon, helium, neon and krypton. Air, excluding water, contains 78 mol% nitrogen, 21 mol% oxygen, 1 mol% argon, and less than 0.05 mol% of each of carbon dioxide, helium, neon and krypton.

The reaction mixture may also comprise methane. It may therefore comprise propane, oxygen and methane. It may also comprise propane, oxygen, inert gas, and methane. It may also consist of propane, oxygen, inert gas and/or methane.

The reaction mixture may also comprise water. It may therefore comprise propane, oxygen and water. It may also comprise propane, oxygen, inert gas, and water. It may also consist of propane, oxygen, inert gas and/or water. The water is preferably present in the reaction mixture in an amount of less than 5 mol%, preferably 0.5 mol% to 4 mol%, preferably 1 mol% to 3 mol%. Including an amount of water in the specified range delivers an increase in the conversion of the reaction without reducing the selectivity for propylene oxide. It therefore contributes to increasing the efficiency of the invention. In a preferred embodiment of the invention, the source of oxygen, inert gas and water in the reaction mixture is air. The reaction mixture may therefore comprise or consist of propane and air. Using water from air in the reaction mixture further improves the efficiency and simplicity of the reaction, as air can be obtained directly from the atmosphere and does not require a further liquification, distillation or drying. The content of water in air typically varies from 0 - 3 mol%, and can easily be controlled by adding further water or drying the air.

In view of the foregoing, the reaction mixture may comprise or consist of the following mol% amounts of propane/oxygen/inert gas/water based upon 100 mol% of the reaction mixture: 40 - 80/5 - 25/15 - 35/0, preferably 50 - 70/10 - 20/20 - 30/0, preferably 60/15/25/0; or 10 - 50/5 - 25/45 - 65/0, preferably 20 - 40/10 - 20/50 - 60/0, preferably 30/15/55/0; or 40 - 80/5 - 25/15 - 35/0 - 5, preferably 50 - 70/10 - 20/20 - 30/1 - 3, preferably 60/15/22/3; or 10 - 50/5 - 25/45 - 65/0 - 5, preferably 20 - 40/10 - 20/50 - 60/1 - 3, preferably 30/15/52/3.

The mole ratio of propane:oxygen in the reaction mixture is preferably 10:1 to 1:2, preferably 5:1 to 1:2, preferably 4:1 to 1:1, preferably 4:1 to 2:1. These ratios lead to an improved selectivity for propylene oxide relative to other products in the reaction, improving the efficiency of the method.

The reaction mixture may be flammable, meaning that it may ignite spontaneously from the heat of the environment without an ignition source. Such spontaneous ignition is called "autoignition". A flammable reaction mixture has an autoignition temperature as defined in ASTM E659.

Prior to step (i), the reaction mixture may be introduced into the reactor. The components of the reaction mixture can be premixed outside of the reactor and added to the reactor together. Alternatively, one or more or all of the components of the reaction mixture may be added separately to the reactor, such that the reaction mixture is formed in the reactor. It is preferred that propane and oxygen (optionally propane and air) are added to the reactor separately.

In order for the reaction mixture to react to form the product mixture, the reaction mixture is heated in the reactor. The heating may be provided by any suitable means and is not particularly limited. In an optional aspect of the invention, the reactor is immersed in a heated salt bath for heating. In the method of the invention, the energy used for heating is preferably provided by renewable energy.

In (i) in the invention, the reaction mixture may be heated to at least 470°C, and preferably the reaction mixture is heated to 470°C to 510°C. It is preferred that the reaction mixture is heated to above the autoignition temperature of the reaction mixture. The autoignition temperature can be determined for the reaction mixture using ASTM E659. By heating the reaction mixture above the autoignition temperature, the yield of propylene oxide can be increased and the efficiency of the method can be improved.

In the method of the invention, the reaction mixture may be heated up in the reactor from room temperature. Alternatively, one or more of the components of the reaction mixture may be heated up prior to their introduction to the reactor. Within the meaning of the present application, room temperature is 20°C. The reaction mixture may be heated at one or more heating rates to a temperature at which the reaction takes place to transform the reaction mixture into the product mixture. The heating rate may be 1 - 10 K/min, optionally 2 - 6 K/min, optionally 2.5 - 5 K/min. A continuous heating rate may be applied between room temperature and the highest temperature reached in the reactor. Alternatively, the heating rate may not be continuous and the temperature may be held at a temperature between room temperature and the highest temperature during the heating stage. For example, in this optional aspect of the invention, if the reaction mixture is being heated to at least 470°C, the temperature may be held at 340 - 360°C, preferably 350°C for more than 5 minutes, optionally more than 15 minutes.

After step (i) and before step (ii), the product mixture may be cooled. The cooling means may be a heat exchanger. During the cooling process, the heat of the product mixture may be used to heat the reaction mixture for a subsequent reaction. This increases the energy efficiency of the process. The cooling may be carried out until room temperature is reached. Alternatively, cooling may be carried out until a suitable temperature is reached for (ii) to be carried out as discussed below. Cooling until (ii) also increases the energy efficiency of the process, by using the heat of the product mixture to carry out (ii). The cooling rate may be 1 - 10 K/min, optionally 2 - 6 K/min, optionally 2.5 - 5 K/min. A continuous cooling rate may be applied between the highest temperature reached in the reactor and the temperature of the product mixture in (ii). Alternatively, in an optional aspect of the invention, the cooling rate may not be continuous and the temperature may be held at a temperature between the highest temperature of the product mixture and the temperature of the product mixture in (ii). If the reaction mixture is being cooled from at least 470°C, the temperature may be held at 340- 360°C, preferably 350°C for more than 5 minutes, optionally more than 15 minutes.

The pressure in (i) in the method of the invention may be at least 1 atmosphere, and is preferably 1 - 5 atmospheres, and is preferably 1 - 3 atmospheres. A lower pressure means more efficient use of resources, as lower pressure requires less energy and less sophisticated equipment.

(i) may be carried out in a continuous method or in a batch method. It is preferred that it is carried out in a continuous method.

The reaction in (i) may be a steady state reaction.

The residence time in the reactor in (i) is not limited. Preferably, the residence time is sufficient so that the proportion of the propane in the reaction mixture that is converted into the product mixture is as specified in the claims. Preferably, the residence time in a batch reaction is 1 hour or less, preferably 30 minutes or less, preferably 5 minutes or less, preferably 1 minute or less.

Preferably, the gas hourly space velocity (GHSV), [$h^{-1}$] in the reactor in a continuous process is 1 to 100,000

$h^{-1}$, 100 to 30,000 $h^{-1}$, 500 to 20,000 $h^{-1}$. Generally for both the batch and continuous processes, a shorter residence time is preferred from the perspective of resource and energy efficiency.

The flow rate of the reaction mixture when a continuous process is used in (i) is also not particularly limited. It may be 1 - 50 mL/min, 2 - 25 mL/min, or 3 - 20 mL/min.

The reactor used in the invention is also not particularly limited, but it must be suitable for carrying out reactions at least up to the temperatures and pressures defined above. The reactor may be a single reactor, meaning that (i) takes place in one reactor only, without requiring multiple reactors. In one embodiment of the invention, the reactor is a quartz reactor. The reactor may also be tubular. It may also have a fixed bed, and be a quartz tubular fixed bed reactor. The reactor may have one or more inlets for supplying one or more components of the reaction mixture. In one optional aspect of the invention, oxidation may be performed in bundles of tubular reactors heated in a salt bath. The tubes may have inner dimensions of 27 mm or less, preferably 25 mm or less.

In the method of the invention, one or more components of the reaction mixture is introduced into the reactor using the one or more inlets. It may also have one or more outlets for removing the product mixture. In the method of the invention, the product mixture may be removed from the reactor using the one or more outlets. Where the reactor has a fixed bed, it may contain an inlet to supply additional oxygen to the bed in case oxygen is used up at that site. Therefore, in the method of the invention, oxygen may be supplied to the fixed bed during the formation of the product mixture.

In the method of the invention, heating the reaction mixture results in oxidation of the propane to form the product mixture comprising propylene oxide. Therefore, in the method of the invention in (i) the oxygen oxidises the propane to deliver propylene oxide. The oxygen itself is essential for the initiation and propagation of the radical chain reactions that lead to the product mixture comprising propylene oxide. It is preferred that the reaction to form the product mixture in (i) takes place in the gas phase. This means that propane, oxygen, and propylene oxide are all preferably gaseous in the conditions in the reactor in (i).

In addition to the propylene oxide, the product mixture may comprise the components of the reaction mixture. Therefore, the product mixture may comprise propylene oxide, propane, and oxygen. If the reaction mixture comprised an inert gas as defined above, this will also be comprised in the product mixture. Therefore, the product mixture may additionally comprise one or more of helium, neon, argon, krypton, xenon, nitrogen and carbon dioxide. If the reaction mixture comprised methane as explained above, this may also be comprised in the product mixture. If the reaction mixture comprised water as explained above, this will also be comprised in the product mixture. In addition, the product mixture may comprise one or more additional products of the oxidation of propane. These additional products may comprise one or more of propylene, ethylene, carbon monoxide, carbon dioxide, water, acrolein, acetaldehyde, and propionaldehyde.

In the method of the invention, the proportion of the propane in the reaction mixture that is converted into the product mixture may be 10 to 60%, 15 to 60%, preferably 25 to 45%, more preferably 25 to 35%, more preferably 28 to 32%. This is determined using the calculation set out in the experimental section below. The amounts of the various components in the product mixture required in the calculation may be determined using the online gas chromatography technique also specified in the experimental section below. The conversions may therefore be determined after (i) and (ii) have been carried out. As an illustration of the meaning of this term, 50% conversion means that a propane content of 30 mol% in the feed at the inlet of the reactor would be reduced to 15 mol% propane at the outlet of the reactor. The conversion can be controlled by changing the amount of oxygen as discussed above, as well as by changing the temperature of the reaction mixture, the flow rate, the GHSV, and the solid material. The propane conversions above are preferred since the propylene oxide selectivity reaches a maximum at a propane conversion of 30%, thus increasing the efficiency of the reaction.

In the method of the invention, the selectivity for propylene oxide may be above 3%, preferably above 5%, preferably above 8%, and is preferably 10 - 12%. This is determined using the calculation set out in the experimental section below. The amounts of the various components isolated from the product mixture required in the calculation may be determined using the online gas chromatography technique also specified in the experimental section below. The selectivity may therefore be determined after (i) and (ii) have been carried out. The selectivity for propylene oxide can be controlled using the propane conversion, which can be controlled by the temperature of the reaction mixture, the flow rate, the GHSV, and the solid material, as discussed above, and can also be controlled using higher amounts of propane in the reactor. This selectivity can also be controlled using the propane to oxygen ratio of the reaction mixture, and the content of the inert gas in the reaction mixture. The efficiency of the reaction is improved when carrying out the reaction to obtain a selectivity in the amounts above.

In the method of the invention, the yield of propylene oxide may be 3% or more, preferably 4% or more. The amounts of the components in the product mixture required for the calculation may be determined using the online gas chromatography technique also specified in the experimental section below. The calculation for the yield is also specified in the experimental section below. The yields may therefore be determined after (i) and (ii) have been carried out. The yield of propylene oxide can be controlled using the propane conversion and selectivity as discussed above. The efficiency

of the reaction is improved when carrying out the reaction to obtain the yields above: higher yields of propylene oxide mean that fewer resources are required relative to the amount of propylene oxide obtained.

In the method of the invention, the yield of carbon dioxide may be less than 4%, preferably less than 3%, preferably less than 2%, preferably less than 1% and most preferably less than 0.6%. The amounts of the components in the product mixture required for the calculation may be determined using the online gas chromatography technique also specified in the experimental section below. The calculation for the yield is also specified in the experimental section below. If carbon dioxide was included e.g. in air in the reaction mixture as described above, this is not taken into account in the carbon dioxide yield calculation. The yield of carbon dioxide can be reduced by increasing the yield of propylene oxide using the measures described above. Achieving yields of carbon dioxide in the amounts specified above satisfies the demand for a method which reduces generation of greenhouse gases.

In the method of the invention, although propylene oxide is the desired product, the selectivity for certain other products in the reaction mixture may also be relevant. This is because some of these other products are also valuable, thus increasing the overall efficiency of the method, as further valuable products can be obtained at the same time as propylene oxide. The selectivity is determined using the calculation set out in the experimental section below. The amounts of the various components isolated from the product mixture required in the calculation may be determined using the online gas chromatography technique also specified in the experimental section below. The selectivity may therefore be determined after (i) and (ii) have been carried out. The selectivity for propylene may be 40 - 85%, and is optionally 45 - 75%. The selectivity for ethylene may be 3 - 20%, and is optionally 5 - 18%. The selectivity for acrolein may be 0.5 - 3%, and is optionally 0.7 - 1.7%. The selectivity for acetaldehyde may be 0.5 - 4%, and is optionally 0.7 - 3%. The selectivity for propionaldehyde may be 0.5 - 2%, and is optionally 0.5 - 1.5%. The selectivity for CO may be 2 - 15%, and is optionally 5 - 12%. The selectivity for propylene can be controlled using the propane conversion: selectivity decreases as the conversion increases. The selectivity for ethylene can also be controlled using the propane conversion: selectivity increases as the conversion increases.

For the same reasons given above, the yield of other products in the reaction mixture may also be relevant. The yield of propylene may be 5 - 25%, preferably 10 - 20%. The yield of ethylene may be 0.1 - 10%, preferably 0.2 - 7%. The yield of CO may be 0.1 - 10%, preferably 0.2 - 7%. The yield of acrolein may be 0.0 - 0.5%. The yield of acetaldehyde may be 0.1 - 2%. The yield of propionaldehyde may be 0.1 - 0.5%. The amounts of the components in the product mixture required for the calculation may be determined using the online gas chromatography technique also specified in the experimental section below. The calculation for the yield is also specified in the experimental section below. The yields may therefore be determined after (i) and (ii) have been carried out. The yield of propylene can be controlled using the conversion of propane discussed above: higher conversion of propane gives higher yield of propylene.

In the method of the invention in (ii) propylene oxide is isolated from the product mixture formed in (i). This means that propylene oxide is separated from any other components in the product mixture to provide propylene oxide which is substantially pure. Substantially pure means that the propylene oxide contains less than 5 wt%, preferably less than 2 wt%, preferably less than 1 wt%, preferably less than 0.1 wt% of components other than propylene oxide. This is determined using the gas chromatography method specified in the experimental section below.

The isolation in (ii) may be carried out by distillation, using the differences in boiling points of the various components in the product mixture to separate them. One distillation column may be used, or more than one distillation columns may be used in series. In a preferred embodiment, fractional distillation is used.

The boiling points of the components in the product mixture at atmospheric pressure are given below:

water: 100°C
acrolein: 53°C
propionaldehyde: 49°C
propylene oxide: 34°C
acetaldehyde: 20°C
propane: -42°C
propylene: -48°C
inert gases: -78°C or lower
ethylene: -104°C
CO: -191.5°C.
Propylene oxide may therefore be isolated from the reaction mixture by distillation.

In this connection, we note that there may be steps between (i) and (ii). The cooling of the product mixture was described above. In addition, the product mixture may be contacted with a drying agent in order to remove water. As mentioned above, cooling may be carried out after (i) until a suitable temperature is reached for (ii) to be carried out. In this case, cooling could be carried out until the temperature of the product mixture is below the boiling point of the product mixture component with the next highest boiling point than propylene oxide. At this point, those product mixture components with higher boiling points than propylene oxide will have condensed and can be separated from the gaseous propylene oxide containing product mixture. This product mixture may then be further cooled to below the boiling point of propylene oxide, but above the boiling point of the component of the product mixture with the next lowest boiling point. At this point, the propylene oxide will condense and can be separated from the gaseous components in order to isolate the propylene oxide. Carrying out (ii) in this way may improve the energy efficiency of the method, as heat from the reaction in (i) is used to carry out the isolation in (ii).

Like (i), (ii) may be carried out in a batch process or in a continuous process. It is preferred that (ii) is carried out in a continuous process, and most preferred that both (i) and (ii) are carried out in a continuous process. This is advantageous from the perspective of efficiency for application on an industrial scale.

In (ii), components in the product mixture other than propylene oxide may also be isolated. This means that these further components may additionally be separated from the product mixture to provide such further components which are substantially pure. Substantially pure for these further components has the same meaning given above for propylene oxide. It is preferred that propylene and ethylene are individually isolated in this way. This is advantageous as it improves the overall efficiency of the process, by isolating more of the valuable products in the product mixture. Propane and oxygen can also be isolated in this manner. This is particularly advantageous as these components can then be recycled by using them in (i) again. In the same way, the inert gases may be separated and recycled. Acrolein, propionaldehyde, CO and acetaldehyde may also be isolated in the same manner. CO may alternatively be isolated using pressure swing adsorption.

In the method of the invention, the reactor may contain a solid material. This means that the solid material is within the reactor and is in contact with the reaction mixture in (i). In this context, "in contact" means that the reaction mixture may touch the solid material. The reaction mixture may flow over the solid material. The solid material may be present in the bed of the reactor. The solid material is different from the reactor itself, and is not part of the structure of the reactor.

The solid material used in the method of the invention is a material which remains in the solid state at the temperatures and pressures used in (i). The solid material is not particularly limited, but it is preferred that it is an inert solid material. This means that it preferably does not react with the reaction mixture in the conditions in (i), or catalyse the formation of the product mixture from the reaction mixture in the reaction in (i). This is preferred because the method delivers propylene oxide simply with solid material which is inert, meaning that this is a simpler way to carry out the method. Using a solid material which is not inert increases the complexity of the reaction taking place in (i) and may lead to a decrease in the yield and selectivity for propylene oxide if it causes decomposition of the propylene oxide. This in turn decreases the overall efficiency of the invention.

The solid material used in the invention may be silicon dioxide ($SiO_2$). Silicon dioxide can take many forms, and may be present as crystalline silica, also known as sea sand. This is preferred as it permits most efficient use of resources, as sea sand is abundant. The silicon dioxide may optionally be quartz wool. It may also be fumed silica, such as amorphous fumed silica. One specific type of amorphous fumed silica which may be used in the invention is Aerosil 380. The solid material may also be silicon carbide (SiC). It may also be boron nitride (BN), optionally hexagonal boron nitride (h-BN). All of these materials are inert solid materials as described above. Other possible solid materials based on boron include elemental boron, $B_4C$, $Ti_2B$, NiB, $Co_2B$, $Co_3B$, $HfB_2$, WB, and edge hydroxylated BN. In the method of the invention, one of these solid materials can be used by themselves, or combinations of two or more of these solid materials can be used.

The specific surface area and pore volume of the solid materials used in the method of the invention is not particularly limited. The specific surface area may be 0.01- 1000 $m^2/g$, and the pore volume may be 0.00001 - 10 $cm^3/g$. The specific surface area of boron nitride may be 1 - 100 $m^2/g$, and the pore volume may be 0.001 - 0.1 $cm^3/g$. The specific surface area of crystalline silicon dioxide may be 0.1 - 10 $m^2/g$, and the pore volume may be 0.0001 - 0.01 $cm^3/g$. The specific surface area of fumed silica may be 1 - 1000 $m^2/g$, and the pore volume may be 0.1 - 10 $cm^3/g$. The specific surface area of SiC may be 0.01 - 1 $m^2/g$, and the pore volume may be 0.0001 - 0.001 $cm^3/g$. The specific surface area of quartz wool may be 0.1 - 10 $m^2/g$, and the pore volume may be 0.0001 - 0.01 $cm^3/g$. The amount of solid material is also not particularly limited. For completeness, we note that the mass of solid material in the reactor does not contribute to the reaction mixture for calculating the relative amounts of components in the reaction mixture discussed above. The specific surface area and pore volume are measured using the methods specified in the experimental section below.

The particle size of the solid materials are not particularly limited. However, the particle size may be within the sieve fraction from 1 to 1000 $\mu m$, preferably 250 to 355 $\mu m$. This applies to silicon carbide (SiC), boron nitride (BN), and silicon dioxide ($SiO_2$) wherein the $SiO_2$ optionally comprises or consists of crystalline silica, or fumed silica, but does not apply to those solid materials not present as particles such as quartz wool.

Without wishing to be bound by theory, it is postulated that the solid material supports mixing of the components in the reaction mixture, and also aids heat transport. However, the similarity in the product distribution over all apparently inert substances suggests that the reaction network itself is identical in all experiments, meaning that it is unlikely that

the solid material acts as a catalyst. This is also supported by the fact that similar reactions occur even without the solid material. Differences between the different filling materials can only be observed with regard to the propane conversion achieved under the same reaction conditions. That the specific solid materials above are inert is a surprising finding, given that surface-stabilized $BO_x$ species were thought to be the active sites in the reaction between propane and oxygen to deliver propylene at the solid-gas interphase (ChemCatChem 9, 3623-3626, (2017)).

To expand further on this point, the invention is based in part on the surprising finding that thermodynamically less stable, oxidation-sensitive products such as propylene oxide can be isolated in industrially relevant yields from oxidation reactions based on fast radical reactions without catalysts. This is surprising since many catalysts have been investigated at high temperatures in oxidation reactions without arriving at the simple solution that valuable products can be obtained without a catalyst. It seems that reaction mechanisms assuming surface reactions are not actually required and that redox-active catalysts that give rise to activated oxygen species on their surface are if anything inappropriate for the formation of sensitive reaction products, such as propylene oxide.

Without wishing to be bound by theory, it is postulated that the thermodynamically limited activation of di-oxygen to superoxide and fast sorption-desorption kinetics may contribute to the surprising effect of the present invention. It is also postulated that prior efforts to control the oxidative potential of atomic oxygen species that can only form at surface defect sites have been less successful, possibly because the required high selectivity requires high sorption specificity and thus long residence times on the surface. As the present invention shows, no such interactions with the surfaces of solid materials are required.

Thus, in the method of the invention, the reactor may contain no solid material as described above. This may be advantageous as it increases the simplicity of the method, as fewer components are required to carry out the method. It also reduces the number of resources required for the method, increasing the efficiency. It is preferred that a catalyst is not present in the reactor, and more particularly it is preferred that a redox-active catalyst is not present in the reactor. For example, it is preferred that no transition metals are present in the reactor. Similarly, it is preferred that none of the following components are present in the reactor: lanthanides; actinides; strong bases including alkali oxides, alkaline earth oxides or basic zeolites; carbon materials including active carbon, carbon nanotubes, graphene, nano-diamond or any other nano-structured carbon materials, wherein the carbon material may be in pure form or modified with P, N or B; all modifications of alumina; and also alkali and alkaline earth phosphates. Where such catalysts, redox-active catalysts, transition metals, or components are present as impurities in a solid material if used, it is preferred that they are present in an amount of less than 0.5wt%, preferably less than 0.2 wt%, preferably less than 0.1 wt%, preferably less than 0.01 wt% based on the total weight of the solid material.

Where the reactor in the method of the invention contains no solid material, this increases the simplicity of the reaction and reduces the amount of resources required for the method, as fewer components are required. Without wishing to be bound by theory, it may also increase the selectivity and yield of the propylene oxide, and therefore the overall efficiency of the method. This is because the presence of a catalyst is not required for the formation of the product mixture, and may even reduce the yield and selectivity if it catalyses further reactions of the propylene oxide in (i).

The contact time of the reaction mixture with the solid material is not particularly limited. It may be 0.1 - 100 g.s/mL, preferably 1 - 10 g.s/mL. The contact time is calculated as described in the experimental section. As mentioned above, the GHSV [$h^{-1}$] in the reactor may be 1 to 100,000 $h^{-1}$, 100 to 30,000 $h^{-1}$, preferably 500 to 20,000 $h^{-1}$. The specified ranges of contact times and GHSV may increase the rate of propylene oxide formation relative to propylene formation and hence the efficiency of the invention. They may also increase the rate of ethylene formation relative to propylene formation.

When solid material is present, the solid material bed depth, which is the depth of solid material in the reactor, is also not particularly limited in the invention. The solid material bed depth may be 1 mm - 100 mm, preferably 2 - 30 mm. Where the solid material is SiC, the bed depth is preferably 15 - 30 mm, from a viewpoint of improving the propane conversion and efficiency of the invention.

As detailed above, the method of the invention is suitable for use in industry. This means that (ii) isolates at least 0.5 kg of propylene oxide, preferably at least 1 kg of propylene oxide, preferably at least 10 kg of propylene oxide, preferably at least 100 kg of propylene oxide. The reaction mixture in (i) may comprise at least 12.5 kg propane, preferably at least 25 kg of propane, preferably at least 250 kg of propane, preferably at least 2500 kg of propane. The space-time yield based on the amount of propylene oxide which can be synthesised using a certain amount of solid material where present over time may be at least 0.02 $g_{propylene\ oxide}/g_{solid\ material}*h$, preferably at least 0.08 $g_{propylene\ oxide}/g_{solid\ material}*h$, preferably at least 0.10 $g_{propylene\ oxide}/g_{solid\ material}*h$. The space-time yield based on the amount of propylene oxide which can be synthesised without solid material over time may be at least 0.005 $g_{propylene\ oxide}/h$, preferably at least 0.02 $g_{propylene\ oxide}/h$.

The method of the invention may further comprise step (iii), (iv), (v), (vi), (vii) or (viii) after (ii). The isolated propylene oxide may be used directly in these reactions. The invention then relates to a method comprising: (i) heating a reaction mixture comprising propane and oxygen in a reactor to form a product mixture comprising propylene oxide; and (ii) isolating the propylene oxide from the product mixture, followed by (iii), (iv), (v), (vi), (vii) or (viii). This means that

the isolated propylene oxide may later be used to produce the following products.

In (iii), the propylene oxide is reacted to produce one or more polyether polyols. This may be achieved by the reaction of the propylene oxide with one or more alcohols or polyols. In an option of the method of the invention, the polyether polyol may be further reacted to produce polyurethane. This may be achieved by reacting the one or more polyether polyols with one or more diisocyanates.

In (iv), the propylene oxide is reacted to produce one or more propylene glycols. This may be achieved by the hydrolysis reaction of the propylene oxide in the presence of an ion exchange resin or acid or alkali. In an option of the method of the invention, the propylene glycol may be further reacted to produce one or more unsaturated polyester resins. This may be achieved by polymerising the one or more propylene glycols with one or more unsaturated diacids or anhydrides, wherein unsaturated diacids are unsaturated organic molecules having two carboxylic acid functionalities such as phthalic acid, isophthalic acid, terephthalic acid. Suitable unsaturated anhydrides contain an anhydride group linked by an unsaturated organic backbone: maleic anhydride is such a compound.

In (v), the propylene oxide is reacted to produce one or more propylene glycol ethers. This may be achieved by the reaction of propylene oxide with an alcohol or phenol. In an option of the method of the invention, the propylene glycol ether is subsequently used to produce paints, inks, and/or coatings comprising the propylene glycol ethers.

In the method of the invention, the isolated propylene oxide may also be reacted with ammonia to produce isopropanolamine in (vi). The isolated propylene oxide may also be reacted with carbon dioxide to produce propylene carbonate in (vii). It may also be isomerized to produce allyl alcohol, acetone, and/or propanal in (viii).

Based on the foregoing, it has unexpectedly been found that it is possible to isolate the sensitive reaction product propylene oxide in acceptable yields by the direct oxidation of propane with oxygen. Propylene oxide has never before been isolated in the oxidative dehydrogenation of propane with oxygen even when using catalysts. The method is also suitable for use on an industrial scale, and makes efficient use of time and resources as it is based on a simple transformation having a minimum number of steps and reagents. The method is also highly efficient as it is based on reactants which can be directly obtained as raw materials. Finally, the method avoids generation of greenhouse gases including carbon dioxide, and also avoids generation of contaminated solvents. Any potential negative environmental impacts of the method are therefore effectively minimized.

## Experimental section

### *Materials and Gases*

All used materials are commercially available and were ordered from the following companies:

$SiO_2$ (Quartz) from Supelco (puriss p.a., Lot number SZBA0210)

Hexagonal boron nitride (*h*-BN) from Alfa Aesar (Quality 99.5 %, Lot number E31M55), Aerosil 380 from Evonik (Lot number 157012015)

Silicon carbide (SiC) from ESK-SiC GmbH (Lot number 654508)

Quartz wool from Carl Roth GmbH (chemically pure, Lot number 067254966 and 208270271) The gases propane (purity 99.95 %), oxygen (purity 99.999 %), helium (purity 99.999 %) and nitrogen (purity 99.999 %) were supplied by the Westfalen company.

MilliPore® water was used.

### *Characterization*

**Nitrogen adsorption** was performed at -196 °C using the Autosorb-6B analyser (Quantachrome) after outgassing the solid materials in vacuum ($SiO_2$ and *h*-BN for 2 h at 200 °C, Aerosil 380 for 12 h at 200°C, SiC for 2 h at 300 °C). All data treatments were performed using the Quantachrome Autosorb software package. The specific surface area $S_{BET}$ was calculated based on DIN ISO 9277:2003-05 "Determination of the specific surface area of solids by gas adsorption using the BET method" using the multipoint Brunauer-Emmett-Teller method (BET) in the p/po = 0.05-0.15 pressure range assuming the $N_2$ cross sectional area of 16.2 $Å^2$.

The total pore volumes were determined based on the method in Chapter 8.4 of the first edition of S. Lowell's "Characterization of porous solids and powders: surface area, pore size and density" by using the amount of physisorbed nitrogen at a relative pressure $p/p_0$ = 0.95. **X-ray fluorescence spectroscopy (XRF)** was used for elemental analysis applying a Bruker S4 Pioneer X-ray spectrometer. For sample preparation, the mixture of 0.1 g of the material and 8.9 g of lithium tetraborate (> 99.995 %, Aldrich) was fused into a disk using an automated fusion machine (Vulcan 2 MA, Fluxana).

**Inductively coupled plasma optical emission spectrometry (ICP-OES)** was used as a second technique for elemental analysis. An Optima 8300 from Perkin Elmer with Zyklon nebulizer was used in axial mode. Minimum two

points calibration with forced intercept at zero were measured with certified standards. Peak evaluation is based on three points per peak. Water was used as spectral blank. Dissolution of the samples was done in a multiwave Pro autoclave from Anton Paar, equipped with teflon liner at 200 °C and 60 bar. Reagents in supra pure quality and water from an ELGA pure water system (VEOLIA) (conductivity 0.05 µS/cm) were used.

**X-ray diffraction (XRD)** patterns were measured on a Bruker D8 ADVANCE II theta/theta diffractometer, using Ni-filtered Cu K$\alpha$ radiation and a position-sensitive LynxEye silicon strip detector. Structure parameters were calculated by least-squares fitting of the diffraction patterns using corresponding structure models taken from the ICSD database. Refinement was performed utilizing the program package TOPAS (version 4.2, copyright 1999-2009 Bruker AXS).

**Propane oxidation** experiments were carried out in a self-built reactor with plug flow characteristics. All measurements were performed at atmospheric pressure and the pressure in the reactor was monitored with pressure sensors upstream and downstream of the reactor tube.

The following general reaction conditions were applied, and the specific conditions are given for the examples below:

Mass of material from 100 to 1500 mg
Temperature from 470 °C to 510 °C
Total flow from 6.7 to 25 ml min$^{-1}$
Propane from 10 to 60 mol%,
Oxygen from 1 to 15 mol%
Helium or nitrogen was used as balance

A certain amount of solid material (sieve fraction from 250 to 355 µm) was filled into the quartz reactor (inner diameter 7 mm) without dilution or the empty reactor was used.

**GHSV** [h$^{-1}$] was calculated using the bulk volume of the material in the reactor $V_{material}$ and the applied volumetric gas flow $\dot{V}_{gas}$ at standard conditions (T=273.15 K and p=0.1013 MPa) according to the following formula:

$$GHSV = \frac{\dot{V}_{gas}}{V_{material}}$$

**Contact** time [g s ml$^{-1}$] was calculated using the mass of material in the reactor and the applied volumetric gas flow at standard conditions (T=273.15 K and p=0.1013 MPa) according to the following formula:

$$contact\ time = \frac{m_{material}}{\dot{V}_{gas}}$$

**Online gas chromatography**

The product gas mixtures were analysed by online gas chromatography (Agilent 7890 GC). The following GC column combinations were used for product analysis:

1.) Plot-Q (length 30 m, 0.53 mm internal diameter, 40 µm film thickness) plus Plot-MoleSieve 5 A (30 m length, 0.53 mm internal diameter, 50 µm film thickness), connected to a thermal conductivity detector (TCD) for analysis of the permanent gases (CO, $CO_2$, and $O_2$) and
2.) Plot-Q (length 30 m, 0.53 mm internal diameter, 40 µm film thickness) plus FFAP (length 30 m, 0.53 mm internal diameter, 1 µm film thickness) connected to a flame ionization detector (FID) for analysis of hydrocarbons and oxygenates.

**Calculation of the propane conversion (*$X_{propane}$*) and selectivity (*$S_i$*)** of product i in percentage, were done based on the carbon number and the sum of all products using the following formulas:

$$X_{C_3H_8} = \frac{\sum_{i=1}^{n} N_i c_i}{\sum_{i=1}^{n} N_i c_i + 3c_{C_3H_8,out}} \cdot 100$$

$$S_i = \frac{N_i c_i}{\sum_{i=1}^{n} N_i c_i} \cdot 100$$

$N_i$ is the number of carbon atoms in product i, $c_i$ is the concentration of product i at the reactor outlet, and $c_{C3H8,out}$ is the propane concentration in the outlet gas.

**Oxygen conversion** was calculated using the following formula, where $c_{O2,in}$ and $c_{O2,out}$ are the concentrations of the oxygen in the feed gas at inlet and outlet position, respectively, of the reactor.

$$X_{O_2} = \frac{c_{O_2,in} - c_{O_2,out}}{c_{O_2,in}} \cdot 100$$

**Yield (*Y*$_i$)** of product i in percentage was calculated by using the following formula:

$$Y_i = \frac{X_{C_3H_8} \cdot S_i}{100}$$

Carbon balance **(*C$_{balance}$*)** was determined as follows:

$$C_{balance} = \frac{\sum_{i=1}^{n} N_i c_i + 3c_{C_3H_8,out}}{3c_{C_3H_8,in}} \cdot 100$$

In all experiments, the carbon balance was 100% +/- 5%. The formation of polymerization products was not observed.

**Reaction rates $r_i$** for propane consumption, propylene formation and propylene oxide formation in mol $g^{-1}$ $h^{-1}$ were calculated using the following formula:

$$r_i = \frac{dn_\mathrm{i}}{d\left(\frac{W}{F}\right)}$$

The amount of starting compound i consumed or product i formed ($n_i$) was used in the unit mol $ml^{-1}$. W is the mass of the solid material ($SiO_2$, h-BN, SiC, Aerosil 380) in g and F is the total flow rate in ml $h^{-1}$.

**Mass transfer limitations** were excluded by measuring the propane conversion when using different amounts of material and different gas flows (see Figure 15) and checked by calculating the dimensionless Mears and Weisz-Prater criteria. $SiO_2$ has the highest Mears modulus of $5.7 \cdot 10^{-6}$ (must be < $1.8 \cdot 10^{-2}$) and Weisz-Prater modulus of $2.24 \cdot 10^{-3}$ (must be < 0.07) for measurements at 490°C of all materials tested, indicating that mass transport limitations do not play a role.

## Examples 1a - 1e

A comparison was made of the performance of the solid material h-BN (example 1a) in propane oxidation with other solid materials, like crystalline silica (sea sand) (example 1b), amorphous fumed silica (Aerosil 380) (example 1c), silicon carbide (example 1d), and quartz wool (example 1e) listed in Table 1. Specific surface area and pore volume vary in a broad range. All materials contain trace impurities of transition metal and main group elements, but the concentration is very low and differs from material to material. These impurities were detected using the elemental analysis techniques above (XRF and ICP-OES).

**Table 1.** Properties of the materials filled into the reactor

| **Example** | $S_{BET}$ ($m^2$ $g^{-1}$) | $V_p$ ($cm^3g^{-1}$) | Impurities (wt-%) | Phase composition |
|---|---|---|---|---|
| | | | 0.7 Si | |

(continued)

| Example | | $S_{BET}$ ($m^2$ $g^{-1}$) | $V_p$ ($cm^3g^{-1}$) | Impurities (wt-%) | Phase composition |
|---|---|---|---|---|---|
| **1a** | h-BN | 9.0 | 0.005 | 0.4 Ca<br>0.2 Zr (0.04) Cr | Hexagonal |
| **1b** | $SiO_2$ | 1.6 | 0.0004 | 0.014 Ti 0.003 Mn | α-quartz |
| **1c** | Aerosil 380 | 394 | 1.8 | - | Amorphous |
| **1d** | SiC | 0.1 | 0.0006 | 0.007 Ti<br>0.002 Mn | mixture of various SiC polytypes<br>main component: moissanite-6H |
| **1e** | Quartz wool | 1.1 | - | - | - |

The propane oxidation reaction was carried out in a tubular fixed bed quartz reactor with plug flow characteristics. The solid materials (sieve fraction from 250 to 355 μm) were filled into the quartz reactor (inner diameter 7 mm) in the absence of further solid materials.

All measurements were performed at atmospheric pressure and the pressure in the reactor was monitored with pressure sensors upstream and downstream of the reactor tube. The following reaction conditions were employed in order to heat a reaction mixture comprising propane and oxygen in the reactor to form a product mixture comprising propylene oxide: Temperature: 470°C to 510°C, wherein the temperature variation was carried out as follows: Using a temperature programme of 2°C/min increase in temperature over time, a temperature in the specified range was reached, which was then maintained for 3.5 hours. During this time the gas phase composition was measured repeatedly by gas chromatography, so that several data points were generated. A mean value was calculated from the points that gave reproducible results within the method's margin of error. Reproducible performance with regard to conversion and selectivity means that the reaction had reached a steady-state.

Feed: $C_3H_8/O_2/He$ = 30/15/55,

Flow rate: 3.3 to 20 ml/min, wherein the variation in the flow rate was examined by performing the reaction at constant temperature in the same way as described with respect to the temperature range above, but by selecting the desired flow rate using the mass flow controllers. These conditions were then maintained for at least 1.5 h and the gas phase was repeatedly analysed by GC to obtain reproducible results.

Mass of solid material h-BN: 0.188 and 0.376 g (contact time: 0.45 to 2.26 g s $ml^{-1}$),
Mass of solid material $SiO_2$: 0.3, 0.6, and 1.0 g (contact time: 0.3 to 8.96 g s $ml^{-1}$),
Mass of solid material SiC: 0.5 g (contact time: 1.5 to 6 g s $ml^{-1}$),
Mass of solid material Aerosil 380: 0.051 g (contact time: 0.15 to 0.61 g s $ml^{-1}$, and
GHSV for h-BN = 686 to 3430 $h^{-1}$
GHSV for $SiO_2$ = 567 to 16920 $h^{-1}$
GHSV for SiC = 930 to 3720 $h^{-1}$
GHSV for Aerosil 380 = 1020 to 4080 $h^{-1}$

The propylene oxide was isolated from the product mixture using gas chromatography. Propane, propylene, ethylene, oxygen and CO were also isolated.

Propylene, propylene oxide, ethylene, and CO are the main products of the reaction. The results are shown in Figure 1, which plots: (a) selectivity for and (b) yield of propylene (filled symbols) and propylene oxide (open symbols); (c) selectivity for ethylene (filled symbols) and CO (open symbols), and (d) conversion of $O_2$ shown as a function of propane conversion. For ease of reference, the results in Figure 1 are also additionally presented individually in figures 2, 3, 4, 5, and 6 for examples 1a to 1e respectively. These figures allow the comparison of the propane conversion that can be achieved by using different materials under identical reaction conditions. The propane conversion differs, but, surprisingly, all materials show identical product selectivity for propylene oxide and propylene at similar propane conversion. Similar product selectivity for ethylene and CO was also found. Furthermore, significant amounts of propylene oxide were formed over simple solid materials such as quartz wool which were expected to be inert.

With increasing propane conversion, the selectivity for propylene decreases, while the selectivity for ethylene and CO increases as can be taken from Figures 1a and 1c. In contrast, Figure 1a shows that the propylene oxide selectivity reaches a maximum of 12% at a propane conversion of 30%. When propylene oxide forms, the oxygen concentration in the gas phase is not zero: Figure 1d shows that not all of the oxygen has been converted at this point. It was completely unexpected that significant yields of propylene oxide can be achieved directly from propane by the method of the invention: as much as 4.4% was delivered (Figure 1b). Propylene was also formed in a yield of 20%. The

yields of the other products were as follows: Ethylene 5.0%, CO 6.3%, $CO_2$ 0.6%, acetaldehyde 1.6%, acrolein 0.5%, and propionaldehyde 0.2%.

Comparing between the results in Figure 1 for the solid materials h-BN, silica, amorphous fumed silica, silicon carbide, and quartz wool, the similarity in the product distributions indicates that the reaction network is identical in all experiments. This is surprising, especially given the information in Table 1 above showing that these solid materials have a range of different surface areas, pore sizes, impurities, and forms. Without wishing to be bound by theory, this indicates that gas-phase reactions may be occurring in the invention instead of surface catalysed reactions. Differences in conversion may be being caused by differences in the heat transfer properties of the materials, or the different materials may have different efficiency in quenching radicals.

Differences between these solid materials can only be observed with regard to the propane conversion, which is achieved under the same reaction conditions.

**Examples 2a - 2c**

This example was carried out in the same manner as example 1b, except that:

Mass of $SiO_2$: 666 mg,
Temperature: 490°C,
Feed in example 2a ($C_3H_8/O_2/He$) = 10/15/75,
Feed in example 2b ($C_3H_8/O_2/He$) = 30/15/55,
Feed in example 2c ($C_3H_8/O_2/He$) = 60/15/25, and
carbon dioxide, acrolein, acetaldehyde, and propionaldehyde were additionally isolated.

In each example, the contact time (W/F) was varied from 1.6 to 6.0 g s $ml^{-1}$, and the conversion of $O_2$ reached 100% at W/F = 6.0 g s $ml^{-1}$ in example 2c.

GHSV for each of these examples = 851 to 3176 $h^{-1}$.

The selectivity for (a) propylene and (b) propylene oxide is shown in Figure 7 as a function of the propane conversion. The selectivity for (a) ethylene and (b) CO is shown in Figure 8. The selectivity for other products which are formed in negligible amounts such as carbon dioxide, acrolein, acetaldehyde, and propionaldehyde is shown in Figure 9. Figure 10 meanwhile shows the ratio of (a) $r_{PO}/r_{propylene}$ and (b) $r_{ethylene}/r_{propylene}$ measured for $SiO_2$ as a function of W/F in these examples (where *"r"* refers to the integral formation rates of the relevant products).

As can be taken from these results, a higher concentration of propane delivers higher propylene oxide selectivity (Figure 7). It also delivers a lower selectivity for propylene, meaning that a higher relative amount of propylene oxide is obtained. This is also demonstrated in Figure 10: an increase in the propane concentration causes an increase in the relative formation rate of propylene oxide (Figure 10a) over propylene. Figure 10b shows the same trend for ethylene. In contrast, the formation of CO and $CO_2$ is hardly affected by the higher propane concentration, as shown by Figures 8b and 9a.

The yields of propylene oxide are highest in the investigated conversion range at inlet concentrations of 30% and 60% propane in examples 2b and 2c (Figure 11). Looking at the yield of C3 products overall, these are highest in the investigated conversion range at an inlet concentration of 30% propane in example 2b (Figure 12).

**Examples 3a - 3c**

These examples were carried out in the same way as example 1a, except that:

672 mg of $SiO_2$ was used as the solid material;
Temperature: 480 - 510°C,
Flowrate: 10 mL/min,
Feed: example 3a used $C_3H_8/O_2/N_2$ 30/15/55,
Example 3b used $C_3H_8/O_2/N_2/H_2O$ 30/15/53.5/1.5, and
Example 3c used $C_3H_8/O_2/N_2/H_2O$ 30/15/52/3.
GHSV = 1259 $h^{-1}$.

The results are shown in figure 13. This depicts (a) selectivity for and (b) yield of propylene (filled symbols) and propylene oxide (open symbols), (c) selectivity for ethylene (filled symbols) and CO (open symbols), and (d) conversion of $O_2$ shown as a function of propane conversion.

These results for example 3a can be compared with those for example 1a in Figure 1. This comparison shows that using nitrogen instead of helium as the balancing gas has no significant impact on the selectivity of the method of

the invention.

Meanwhile, increasing the amount of water in examples 3b and 3c surprisingly delivers an increase in the conversion of propane, as can be taken from figure 13 where the propane conversion is largest for the data points for example 3c. This has no significant impact on the selectivity of the method of the invention.

The fact that the reaction works in the presence of nitrogen and water is a valuable finding which permits the use of air as an oxygen source in the invention.

**Examples 4a and 4b**

These examples were carried out in the same manner as example 1b and 1d respectively, except that:

Temperature: 490°C,
Flow: 10 ml $min^{-1}$,
the material bed length was varied from 1.7 - 27.6 mm for SiC and 1.8 - 18.4 for $SiO_2$,
and only the propane conversion was measured.

The results are shown in Figure 14. The propane conversion increases with increasing layer height of the material, but the relationship is not linear.

**Examples 5a and 5b**

These examples were carried out in the same manner as example 1b and 1d respectively, except that:

Temperature: 490°C,
Flow was varied in the range of 4 to 25 ml $min^{-1}$,
Mass of solid material $SiO_2$: 0.100, 0.333, 0.666, and 1.000 g,
Mass of solid material SiC: 0.100, 0.250, 0.500, 1.000, and 1.500 g.

Mass transfer limitations in the process of the invention were excluded by measuring the propane conversion when using different amounts of material and different gas flows. The results are shown in Figure 15. This conclusion was checked by calculating the dimensionless Mears and Weisz-Prater criteria. $SiO_2$ has the highest Mears modulus of $5.7 \cdot 10^{-6}$ (must be < $1.8 \cdot 10^{-2}$) and Weisz-Prater modulus of $2.24 \cdot 10^{-3}$ (must be < 0.07) for measurements at 490°C of all materials tested, indicating that mass transport limitations do not play a role.

**Examples 6a and 6b**

These examples were carried out in the same manner as example 1b and 1d respectively, except that:

Flow was 10 ml $min^{-1}$,
Mass of solid material $SiO_2$ in 6a: 0.4881 g,
Mass of solid material SiC in 6b: 0.5002 g.

These experiments were used to generate the data in figure 16.

A limitation of the reaction rate by film diffusion can be excluded in the present experiments, as can be taken from Figures 15 and 16. In this context, the film represents a dense layer of species adsorbed on the surface of the catalyst. A reaction rate which is limited by film diffusion means that the transport of reactants to the surface and the transport of products from the surface to the gas phase through the film is slower than the reaction at the catalyst active site. In such a case, the macroscopically measured rate is the rate of diffusion and not the rate of the reaction. This can be excluded in this case since lower conversion at lower total flow rates would be observed: figure 15 shows that this is not the case. Similarly, Figure 16 would show a curve shape if film diffusion limited the reaction rate. Instead linear behaviour suggests that the reaction rate is not limited by film diffusion. Without wishing to be bound by theory, this supports the hypothesis that the solid material does not behave as a catalyst in the invention.

Figure 15a shows that the 600mg $SiO_2$ systems delivers a higher conversion than 1000mg. Once again without wishing to be bound by theory, it is postulated that the presence of the solid material may even have a negative effect on the conversion, perhaps due to radical quenching of the reaction mixture by the solid material.

The rates of propane conversion were used to calculate the apparent activation energies ($E_a$) of propane consumption, as shown in Table 2 below. The activation energies are much higher than those of vanadium oxide catalysts most commonly studied in propane oxidation, where they range from 40 to 170 kJ/mol (ACS Catal. 4, 3357-3380, (2014)).

The data agree with values measured previously in concentrated feeds over boron nitride (Science 354, 1570-1573, (2016) and Ind. Eng. Chem. Res. 58, 2170-2180, (2019)). In oxygen-rich feed, on the other hand, values around 190 kJ/mol were measured (ChemCatChem 9, 1788-1793, (2017), Chem. Eng. Sci. 186, 142-151, (2018), and Chem. Commun. 56, 9882-9885, (2020)). The high values indicate that the reactions take place in the gas phase.

**Table 1.** Apparent activation energies

| | $E_{a,propane}$ (kJ $mol^{-1}$) | Feed composition ($C_3H_8/O_2$/Inert) |
|---|---|---|
| h-BN | 230 | 30/15/55 |
| $SiO_2$ | 286 | 30/15/55 |
| Aerosil 380 | 269 | 30/15/55 |
| SiC | 323 | 30/15/55 |
| Quartz wool | 297 | 30/15/55 |
| BNOH | 184 | 1/1.5/3.5 |
| h-BN | 192 | 1/1/9 |
| h-BN | 180 | 1/1.5/3.5 |
| h-BN | 253 | 30/15/55 |

Because of the non-microporous nature of the solid materials, rate limitations due to pore diffusion are unlikely. Thus, without wishing to be bound by theory, the results above suggest that the surface of the solid materials does not directly catalyse the reaction.

**Reference Examples 1a - 1c**

Temperature-programmed experiments were carried out to contribute to a mechanistic understanding of the reaction. The experiments were performed in the same reactor setup as example 1.

An online mass spectrometer (QMA 400, Pfeiffer Vacuum) was used for recording the reactant and product gas streams. In reference example 1a, 670 mg of $SiO_2$ was loaded into the reactor and in reference example 1b 665 mg of h-BN was loaded. No solid material was used in reference example 1c. For these reactions:

Flow: 10 ml/min
Feed: 30 % propane, 15 % oxygen and 55 % helium thus corresponding to a flow of 3.0 mL/min propane, 1.5 mL/min oxygen and 5.5 mL/min helium.

Temperature: the reactor was heated up from room temperature to 350°C with a heating rate of 5 K $min^{-1}$ and held at this temperature for a minimum of 15 minutes. Then the temperature-programmed experiment was performed by heating up to 490°C with a heating rate of 2.5 K $min^{-1}$. The temperature was then held at 490 °C for 1 hour before cooling using the same temperature profile used for heating but in reverse: cooling at 2.5 K $min^{-1}$ to 350°C, holding at this temperature for a minimum of 15 minutes, then cooling at 5 K $min^{-1}$ to room temperature.

The reaction gas was withdrawn approximately 5 cm behind the material bed by using a capillary-vacuum pump combination and fed into the mass spectrometer. All m/z from 18 to 60 were monitored simultaneously with a scanning rate of 50 ms per m/z.

Figure 17 shows the temperature program (start of the experiment at the bottom) and normalized signals of all mass to charge ratios (m/z) from 18 to 60 as a function of temperature and time in a contour plot for reference example 1a. Figure 17 clearly shows that the gas composition does not change over a wide temperature range (350 °C to approx. 470 °C). At 470°C, the reaction ignites with the sudden formation of propylene (increase in m/z 42) and the consumption of propane (decrease in m/z 44). After the reaction reaches its full extent, products such as water and propylene oxide are observed as molecular ion peaks (increase in m/z of 18 and 58, respectively). At the same time, the m/z range from 25 to 27 also shows increased intensity, which could be attributed to the formation of ethylene or molecular fragments of formed propylene. The consumption of oxygen can be clearly seen in the decreasing intensity of m/z 32.

These results for reference example 1a in figure 17 indicate that propylene oxide and propylene are most efficiently formed after ignition of the propane-oxygen reaction mixture. Without wishing to be bound by theory, the ignition may initiate the corresponding gas-phase reactions.

The ignition behaviour differs only slightly when boron nitride is filled into the reactor instead of $SiO_2$ in reference example 1b: see figure 18. Meanwhile, the results in Figure 19 for the reaction without any solid material in reference example 1c suggest that filling the reactor is not needed to produce propylene oxide using the method of the invention.

Without wishing to be bound by theory, it is postulated that the solid material supports gas mixing and heat transport, but is not necessary for product formation. Further, it is also postulated that since there is no redox-active catalyst in the reactor which can lead to subsequent reactions of less stable products such as propylene oxide with the oxygen that is still present, total oxidation of these valuable products to $CO_2$ is reduced.

**Reference example 2**

This example was carried out in the same way as reference example 1a, but for heating and cooling between 350°C and 490°C, 5 K $min^{-1}$ was used instead.
The results are shown in figure 20. The ignition behaviour differs only slightly when the heating rate is increased as can be taken from a comparison of figure 20 with figure 17.

**Reference example 3**

This example was carried out in the same way as reference example 1a, except that 672 mg of $SiO_2$ was used. The feed composition was also changed to 10 % propane, 5 % oxygen and 85 % helium thus corresponding to a flow of 1.0 mL/min propane, 0.5 mL/min oxygen and 8.5 mL/min helium. The results are shown in figure 21. These indicate that no ignition occurred. Conversion of propane and oxygen are only small, and propylene oxide, water, and propylene were only formed in small amounts.

**Reference example 4**

The economic viability of a hypothetical one-step process was estimated using Aspen HYSYS. Figure 22 shows the flowchart of the direct process (40 % propane conversion at 490 °C and 1 bar, 11 % propylene oxide selectivity) and compares this with current processes for synthesising propylene oxide, such as the Oleflex process followed by the HPPO process. As can be taken from figure 22, the process of the invention compares favourably with the current processes for manufacturing propylene oxide, so the direct process of the invention for producing propylene oxide from propane definitely has potential for industrial application. In particular, the known Oleflex process shown in Figure 22 requires higher temperature and pressure conditions than the present method, but does not even deliver propylene oxide. To transform the propylene obtained from the Oleflex process into propylene oxide using the HPPO process, a further step is required, significantly reducing the efficiency and increasing the time and resources required to obtain the propylene oxide.
The yield of 10.3-13.3% for the HPPO process in figure 22 is relative to propane to provide a direct comparison with the invention. Although the yield of the HPPO process is somewhat higher than the method of the invention, in addition to the two steps required as discussed above, the oxidant hydrogen peroxide has to be produced for the HPPO process which requires further resources. In contrast, the present invention can be carried out in a single step with readily available air as the oxidant.
As also shown in figure 22, MTBE and Styrene are valuable co-products obtained from the Oleflex process. Figure 22 also shows that significant amounts of propylene, CO and ethylene are also produced by the present invention: these are also valuable products.
The present method is the basis for the Aspen simulation model shown in Figure 23. The ASPEN programme was used to estimate the cost of propylene oxide made using the invention and came up with a reasonable price which is already competitive with the market price, even though the process of the invention has not been optimised for the simulation in the same way that an industrial method would be optimized. These prices reflect the overall high efficiency of the present method.
Moreover, the results show that the process can be absolutely green if the energy supply is provided by renewable energy, because hardly any climate-damaging $CO_2$ is produced as a by-product. It should also be mentioned that the efficiency of the method of the invention can easily be further improved by making use of heat produced in the process, as well as by using air instead of a helium-oxygen-mixture as feed gas as demonstrated by example 3 above. Efficiency can also be improved by optimizing the distillation process for isolating the propylene oxide.

**Claims**

**1.** A method for producing propylene oxide, the method comprising:

(i) heating a reaction mixture comprising propane and oxygen in a reactor to form a product mixture comprising propylene oxide; and

(ii) isolating the propylene oxide from the product mixture.

2. The method according to Claim 1, wherein the reaction mixture is heated to at least 470°C under 1 - 5 atmospheres, and preferably wherein the reaction mixture is heated to 470°C to 510°C under 1 - 3 atmospheres.

3. The method according to any of Claim 1 or 2, wherein the propane is present in the reaction mixture in an amount of 5 mol% to 80 mol%, preferably 20 mol% to 70 mol%, based upon 100 mol% of the reaction mixture.

4. The method according to any of Claims 1, 2, or 3, wherein a) the oxygen is present in the reaction mixture in an amount of 5 mol% to 25 mol%, preferably 12 mol% to 18 mol%, based upon 100 mol% of the reaction mixture; and/or b) the reaction mixture comprises an inert gas, wherein the inert gas is preferably present in the reaction mixture in an amount of 5 mol% to 90 mol%, preferably 20 mol% to 70 mol%, preferably 25 mol% to 65 mol%, based upon 100 mol% of the reaction mixture.

5. The method according to any of Claims 1, 2, 3, or 4, wherein the mole ratio of propane to oxygen in the reaction mixture is 10:1 to 1:2.

6. The method according to any of Claims 1, 2, 3, 4, or 5, wherein the proportion of the propane in the reaction mixture that is converted into the product mixture is 10 to 60%, preferably 25 to 45%.

7. The method according to any of Claims 1, 2, 3, 4, 5, or 6, wherein the selectivity for propylene oxide is above 3%, preferably above 5%.

8. The method according to any of Claims 1, 2, 3, 4, 5, 6, or 7, wherein the yield of propylene oxide is 3% or more.

9. The method according to any of Claims 1, 2, 3, 4, 5, 6, 7, or 8, wherein the yield of carbon dioxide is less than 4%.

10. The method according to any of Claims 1, 2, 3, 4, 5, 6, 7, 8, or 9, wherein the reactor contains a solid material and the reaction mixture is in contact with the solid material.

11. The method according to Claim 10, wherein the solid material comprises at least one of silicon carbide (SiC), boron nitride (BN), and silicon dioxide ($SiO_2$) wherein the $SiO_2$ optionally comprises or consists of crystalline silica, quartz wool or fumed silica.

12. The method according to any of Claims 1, 2, 3, 4, 5, 6, 7, 8, 9, 10, or 11, wherein a redox-active catalyst is not present in the reactor.

13. The method according to any of Claims 1, 2, 3, 4, 5, 6, 7, 8, 9, 10, 11, or 12, wherein

the reactor is a single reactor, and/or
the reactor is a tubular fixed bed reactor.

14. The method according to any of Claims 1, 2, 3, 4, 5, 6, 7, 8, 9, 10, 11, 12, or 13, further comprising introducing the reaction mixture comprising propane and oxygen into the reactor before (i).

15. The method according to any of Claims 1, 2, 3, 4, 5, 6, 7, 8, 9, 10, 11, 12, 13, or 14 further comprising (iii), (iv), (v), (vi), (vii), or (viii) after (ii):

(iii) reacting the propylene oxide to produce one or more polyether polyols, and optionally further reacting the one or more polyether polyols to produce one or more polyurethanes; or
(iv) reacting the propylene oxide to produce one or more propylene glycols, and optionally further reacting the one or more propylene glycols to produce one or more unsaturated polyester resins; or
(v) reacting the propylene oxide to produce propylene glycol ethers, and optionally producing paints, inks, and/or coatings comprising the propylene glycol ethers; or
(vi) reacting the propylene oxide to produce isopropanolamine; or
(vii) reacting the propylene oxide to produce propylene carbonate; or
(viii) reacting the propylene oxide to produce allyl alcohol, acetone, and/or propanal.

Figure 1.

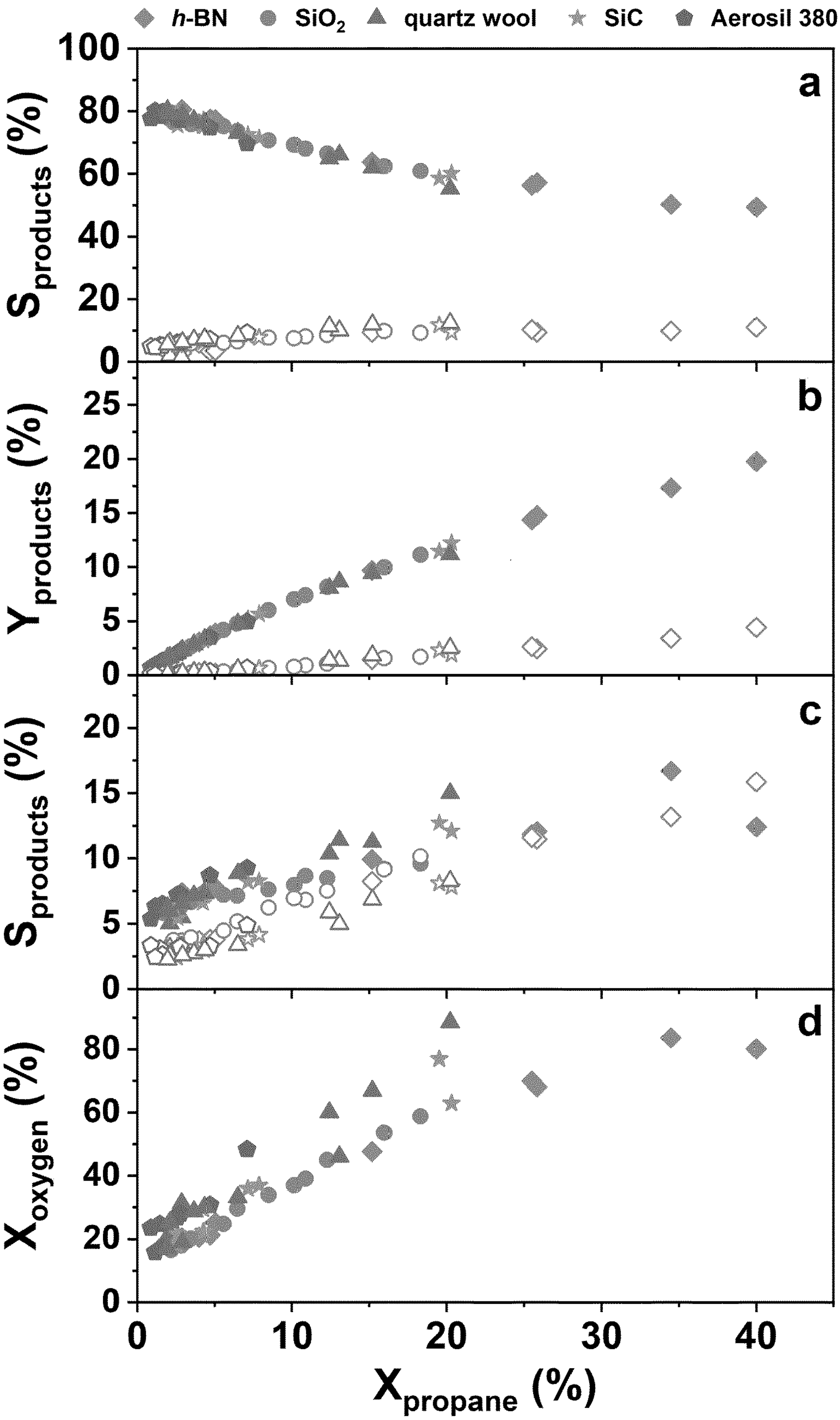
h-BN
SiO2
quartz wool
SiC
Aerosil 380
a
b
c
d
Sproducts (%)
Yproducts (%)
Sproducts (%)
Xoxygen (%)
Xpropane (%)

Figure 2.

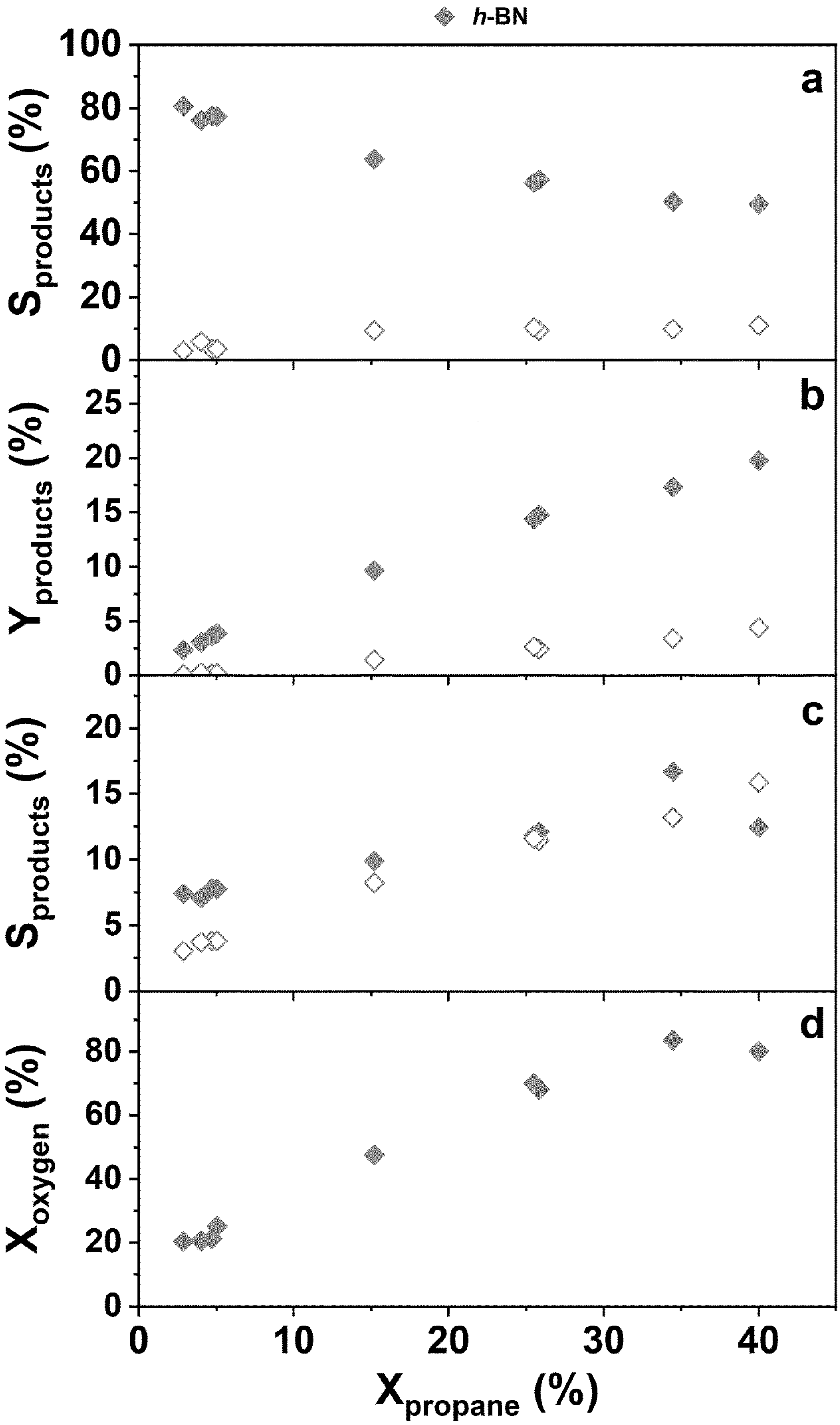
h-BN
a
b
c
d
$S_{products}$ (%)
$Y_{products}$ (%)
$S_{products}$ (%)
$X_{oxygen}$ (%)
$X_{propane}$ (%)
0
20
40
60
80
100
5
10
15
20
25
0
10
20
30
40

Figure 3.

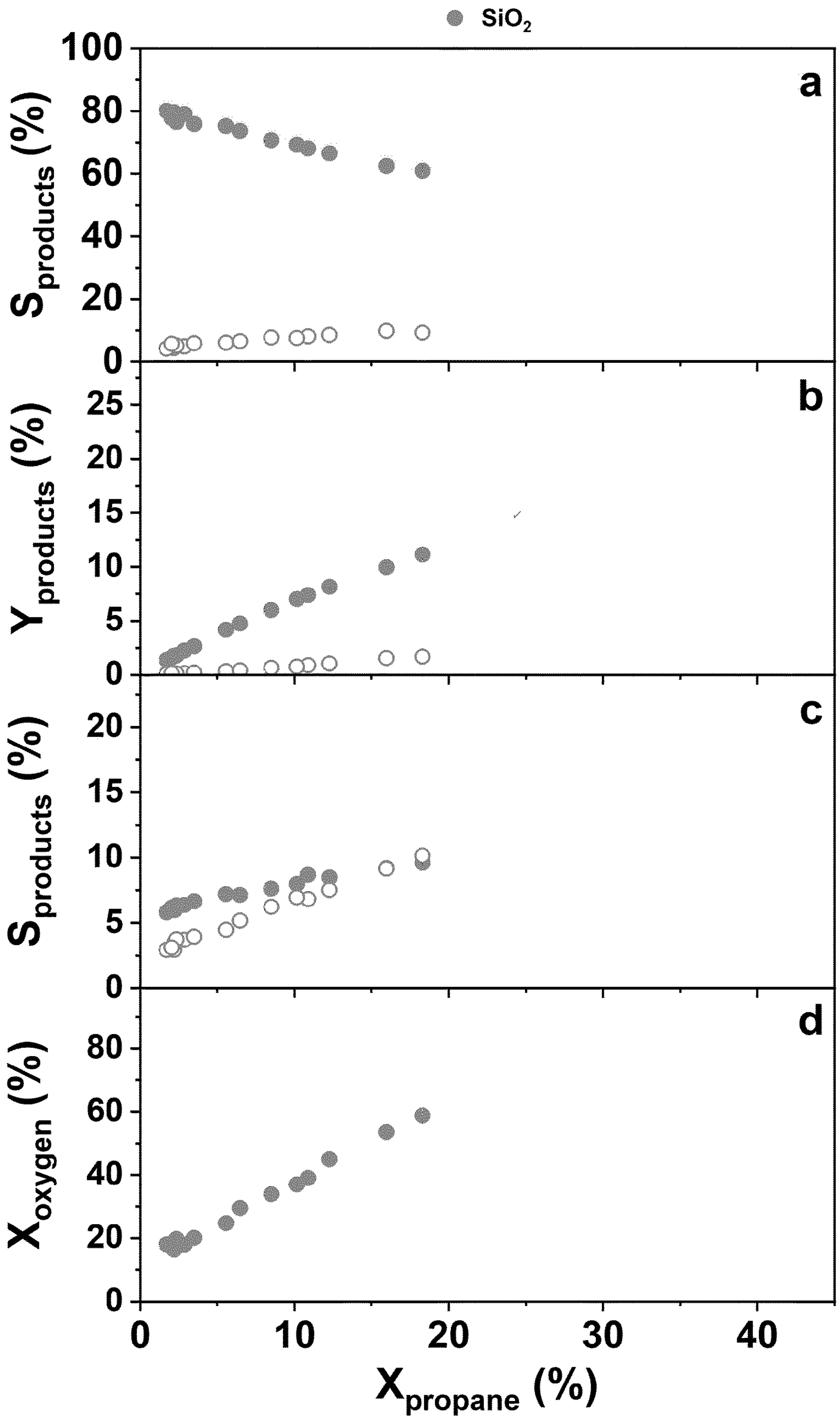
SiO2
a
b
c
d
Sproducts (%)
Yproducts (%)
Sproducts (%)
Xoxygen (%)
Xpropane (%)
100
80
60
40
20
0
25
20
15
10
5
0
10
20
30
40

Figure 4.

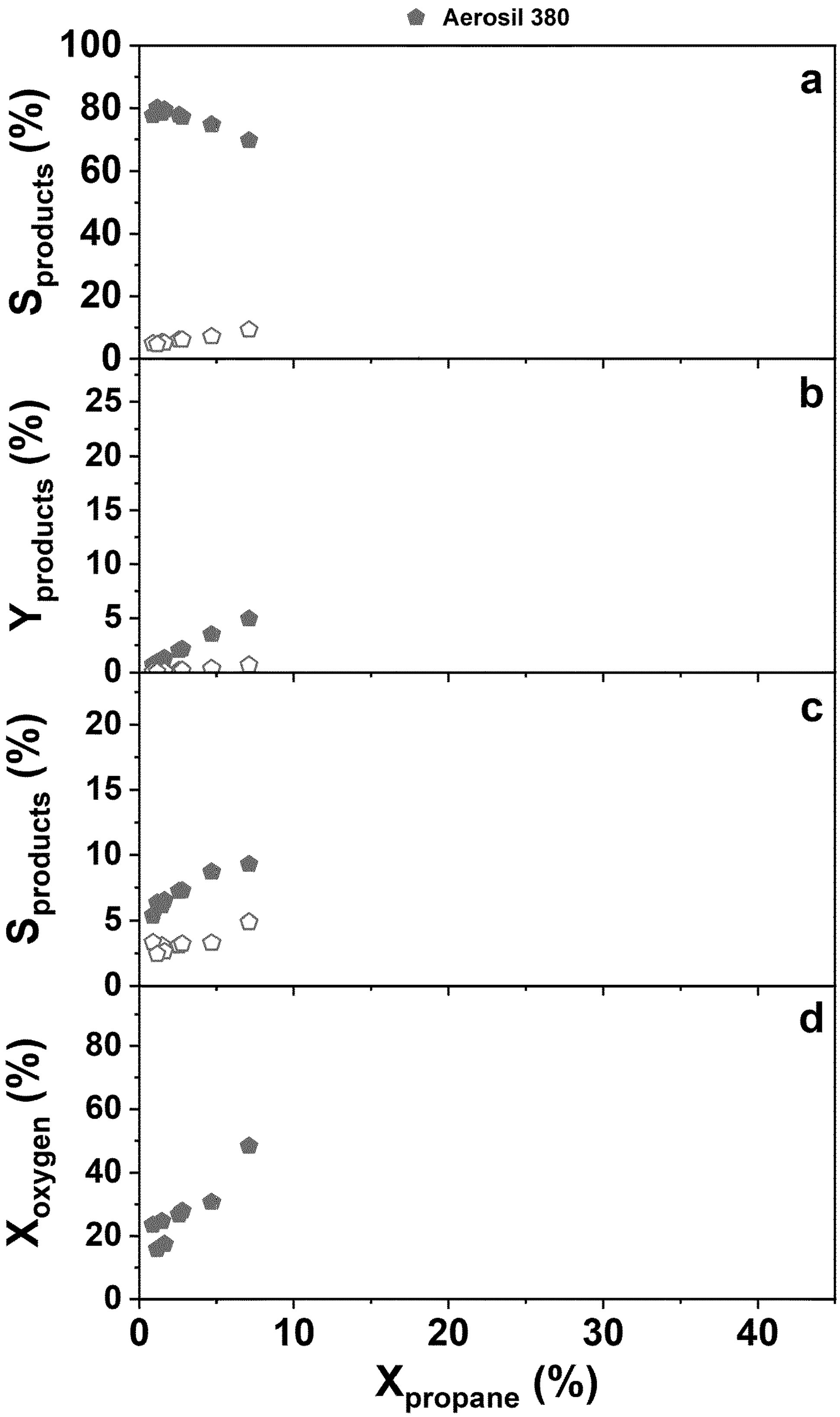
Aerosil 380
a
b
c
d
$S_{products}$ (%)
$Y_{products}$ (%)
$S_{products}$ (%)
$X_{oxygen}$ (%)
$X_{propane}$ (%)
0
20
40
60
80
100
0
5
10
15
20
25
0
5
10
15
20
0
20
40
60
80
0
10
20
30
40

Figure 5.

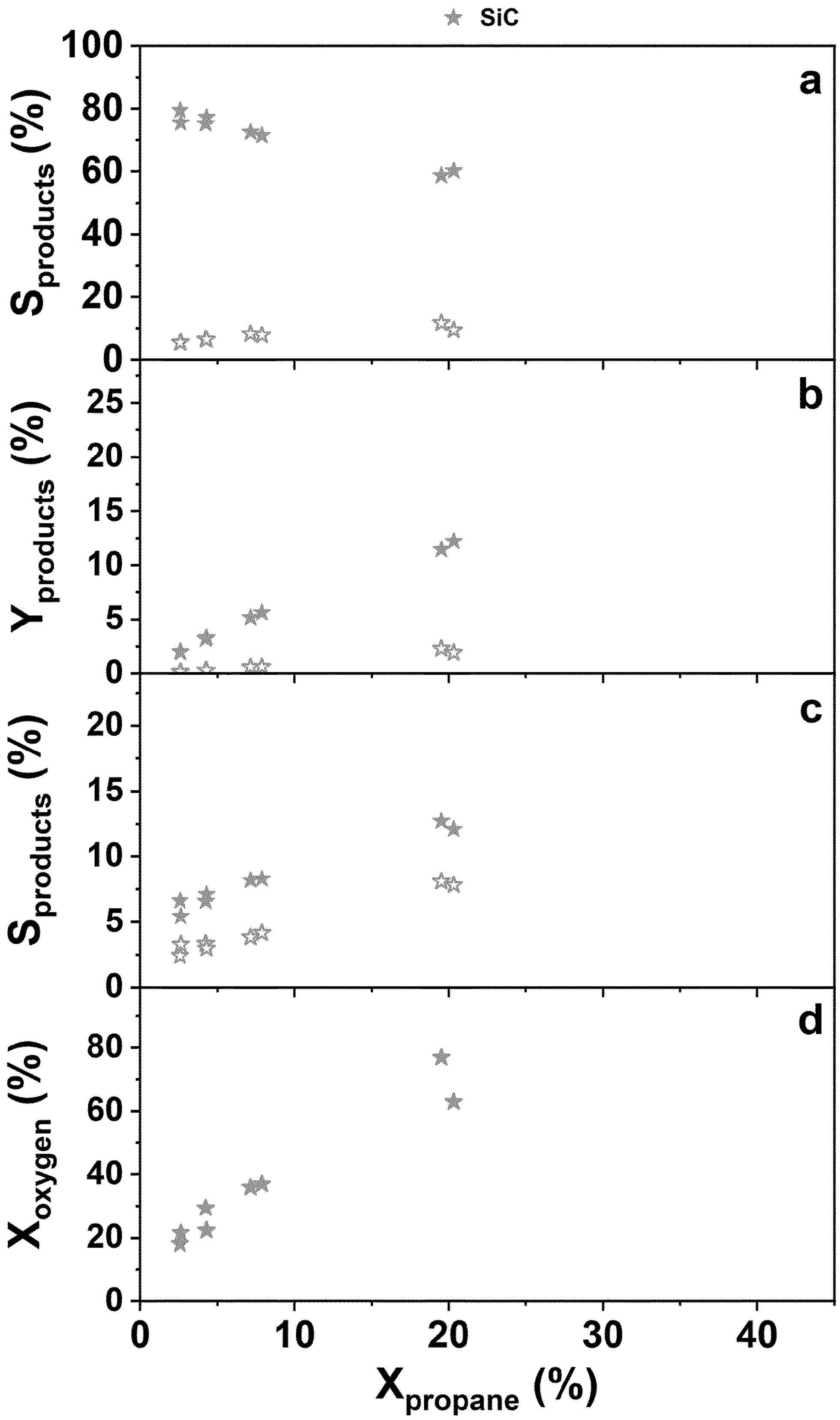
SiC
a
b
c
d
$S_{products}$ (%)
$Y_{products}$ (%)
$S_{products}$ (%)
$X_{oxygen}$ (%)
$X_{propane}$ (%)
0
20
40
60
80
100
0
5
10
15
20
25
0
5
10
15
20
0
20
40
60
80
0
10
20
30
40

Figure 6.

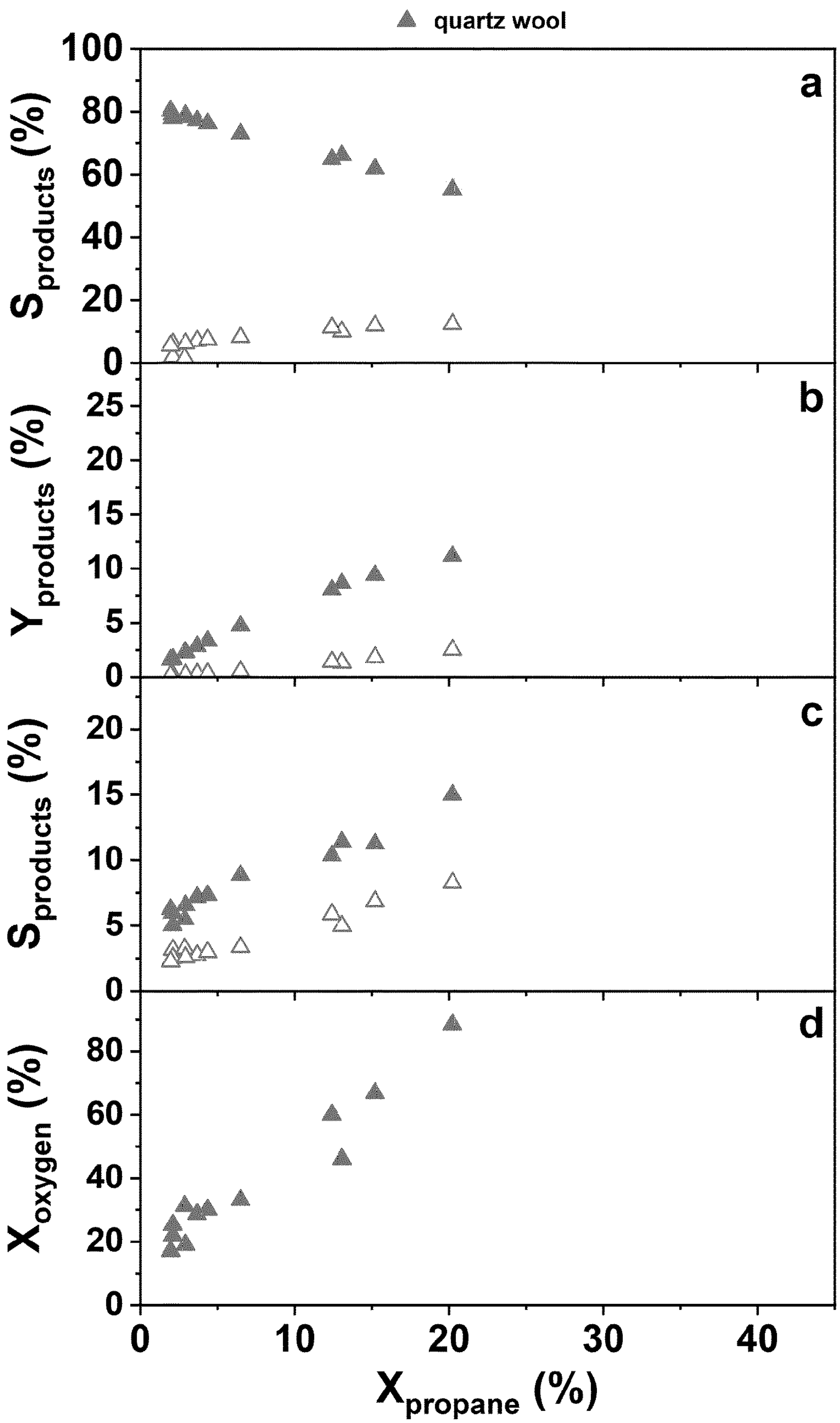
quartz wool
a
b
c
d
$S_{products}$ (%)
$Y_{products}$ (%)
$S_{products}$ (%)
$X_{oxygen}$ (%)
$X_{propane}$ (%)
100
80
60
40
20
0
25
20
15
10
5
0
20
15
10
5
0
80
60
40
20
0
0
10
20
30
40

**Figure 7.**

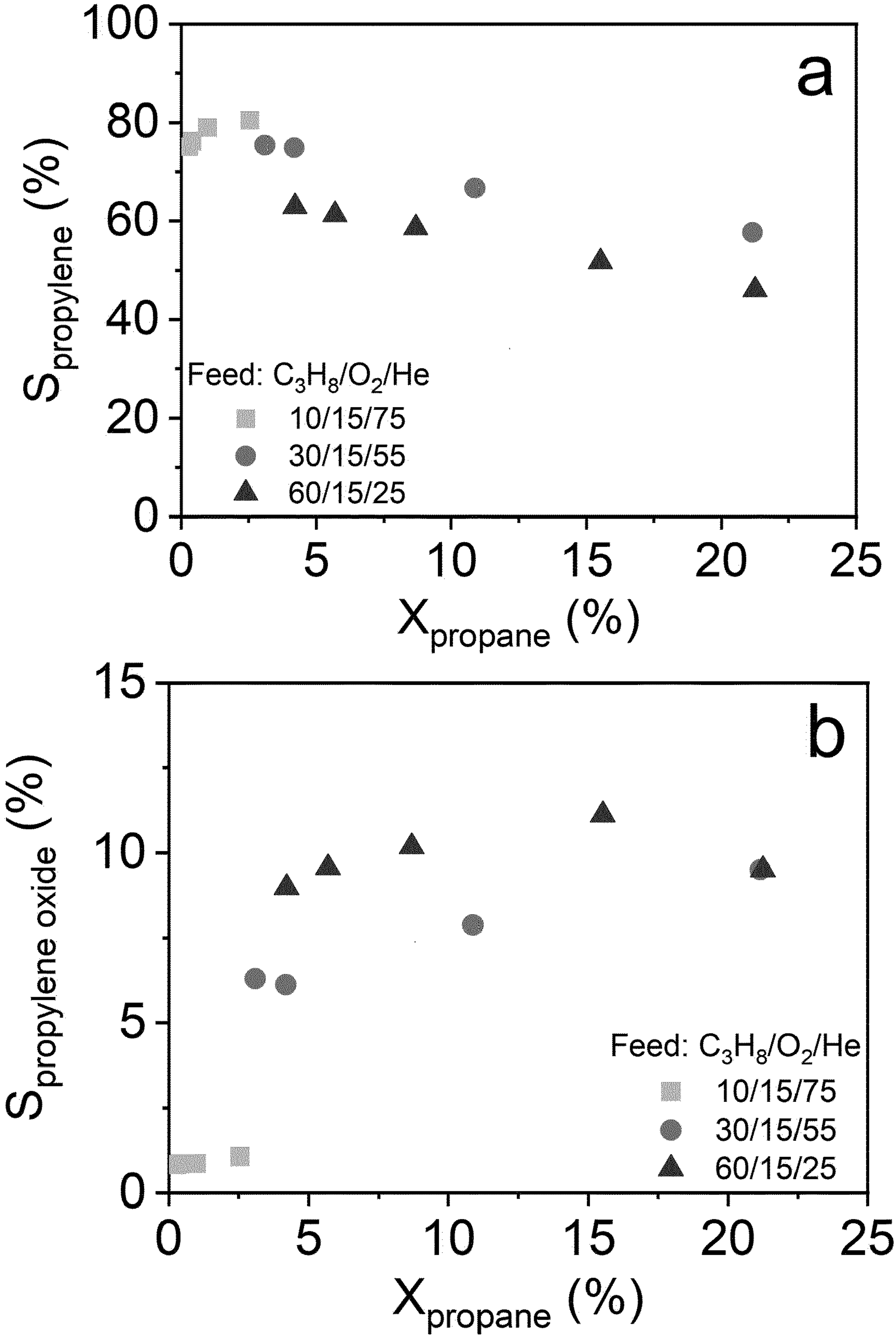
a
Feed: $C_3H_8/O_2/He$
10/15/75
30/15/55
60/15/25
$S_{propylene}$ (%)
$X_{propane}$ (%)
b
Feed: $C_3H_8/O_2/He$
10/15/75
30/15/55
60/15/25
$S_{propylene\ oxide}$ (%)
$X_{propane}$ (%)

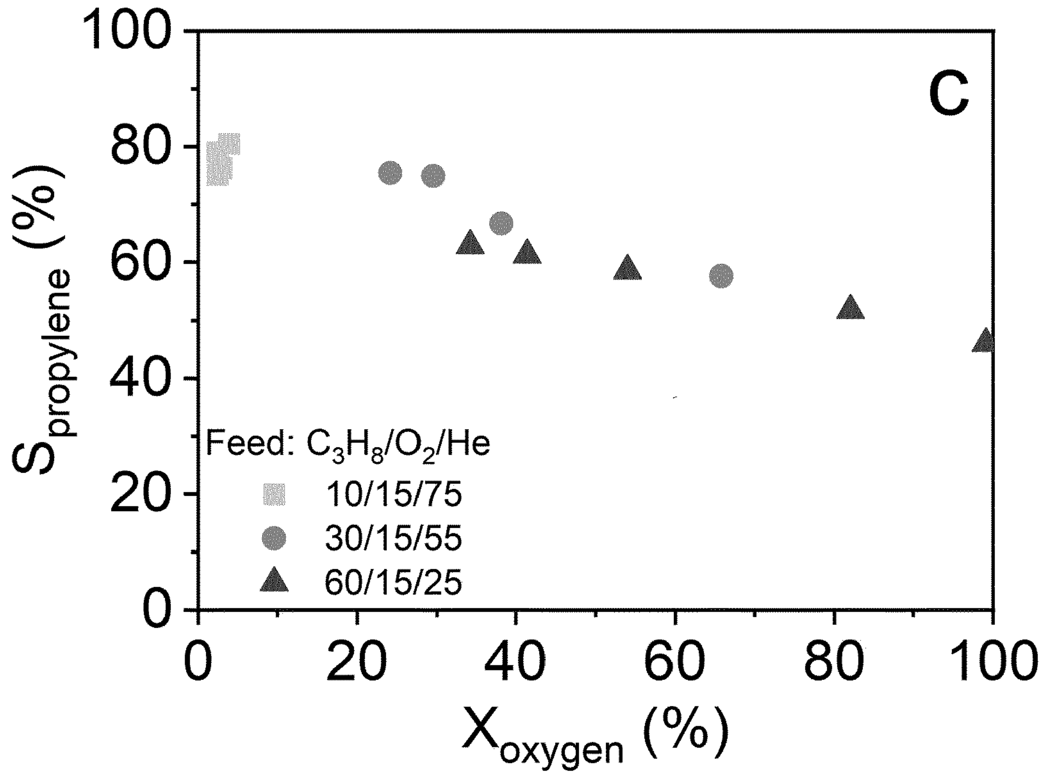
c
$S_{propylene}$ (%)
$X_{oxygen}$ (%)
Feed: $C_3H_8/O_2/He$
10/15/75
30/15/55
60/15/25
0
20
40
60
80
100

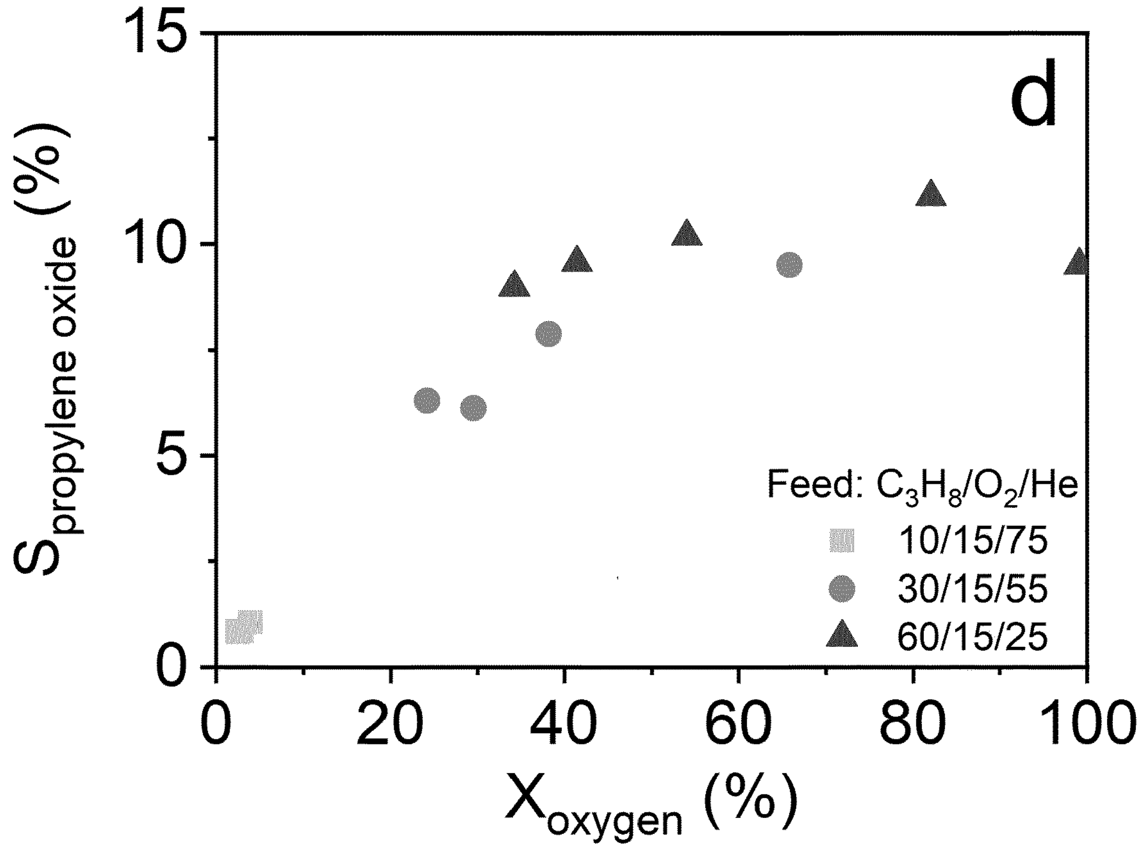
d
$S_{propylene\ oxide}$ (%)
$X_{oxygen}$ (%)
Feed: $C_3H_8/O_2/He$
10/15/75
30/15/55
60/15/25
0
5
10
15
0
20
40
60
80
100

**Figure 8**

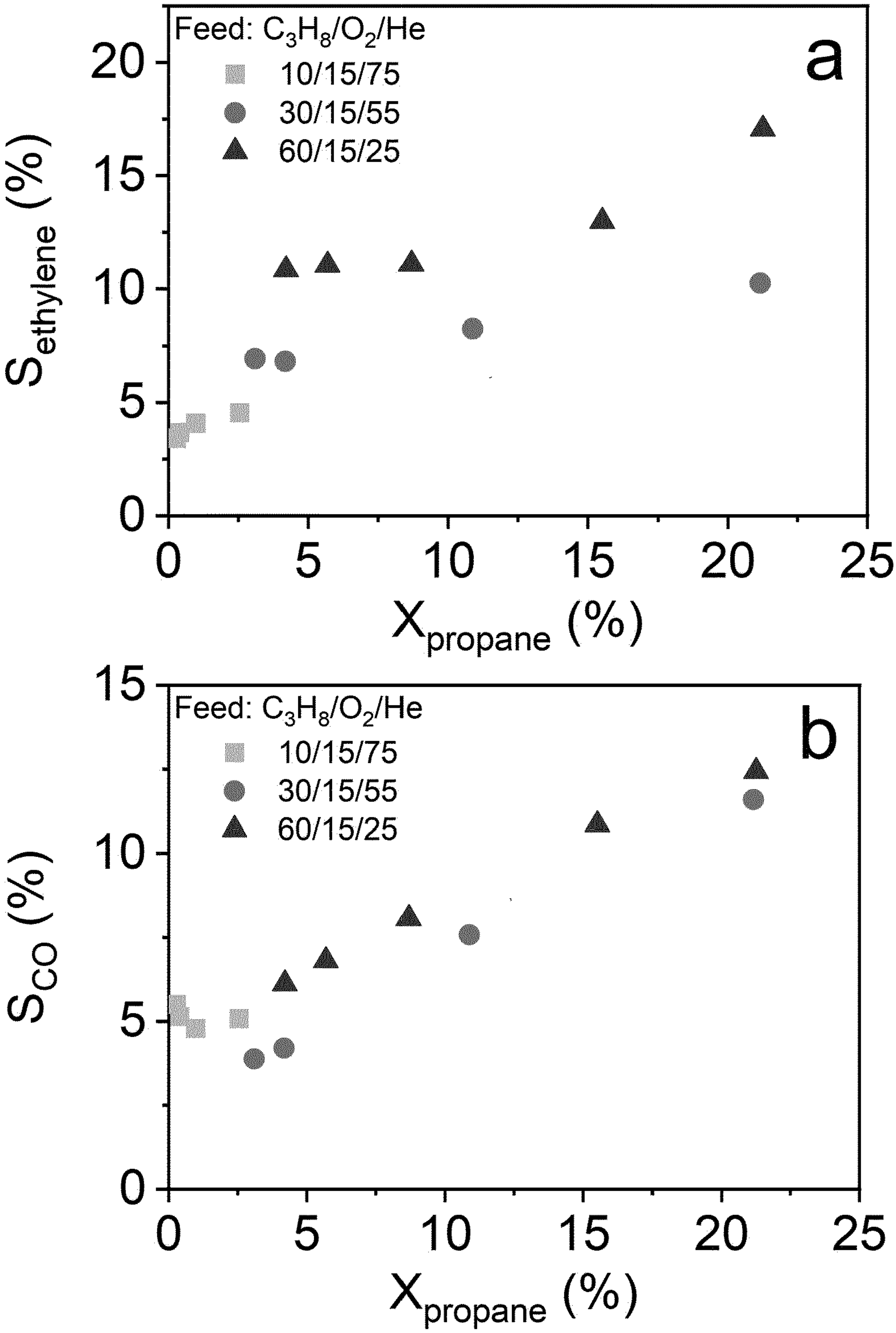
Feed: $C_3H_8/O_2/He$
10/15/75
30/15/55
60/15/25
a
$S_{ethylene}$ (%)
$X_{propane}$ (%)
0
5
10
15
20
25
Feed: $C_3H_8/O_2/He$
10/15/75
30/15/55
60/15/25
b
$S_{CO}$ (%)
$X_{propane}$ (%)

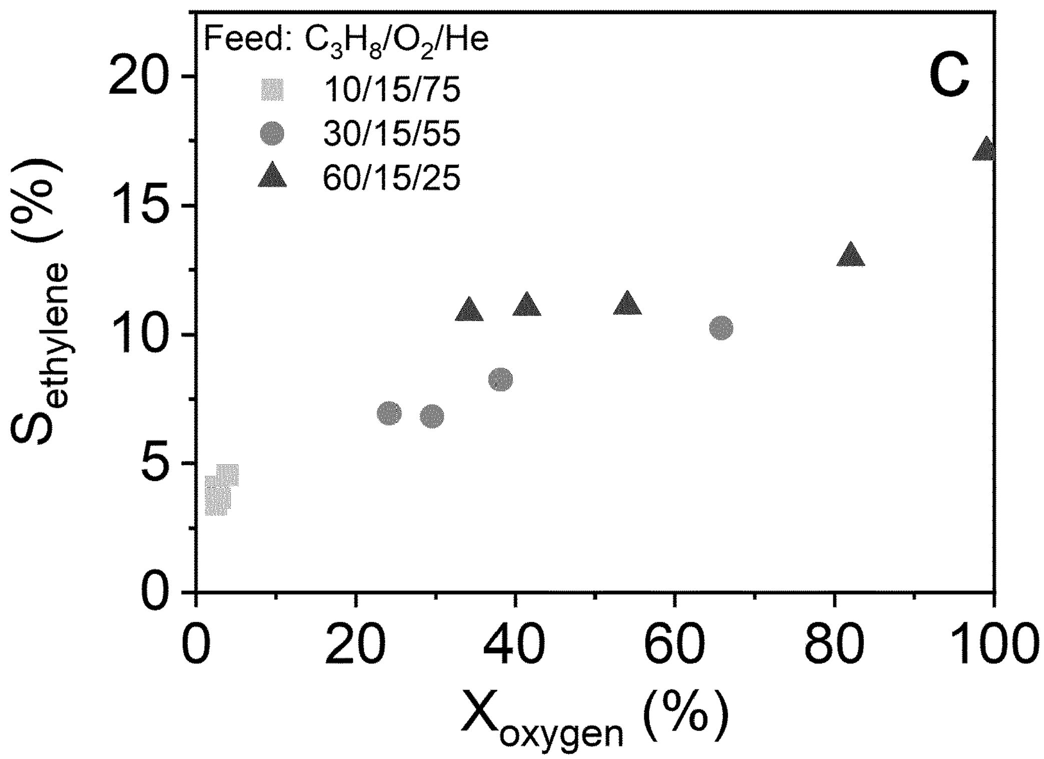
Feed: $C_3H_8/O_2/He$
10/15/75
30/15/55
60/15/25
c
$S_{ethylene}$ (%)
$X_{oxygen}$ (%)
0
5
10
15
20
0
20
40
60
80
100

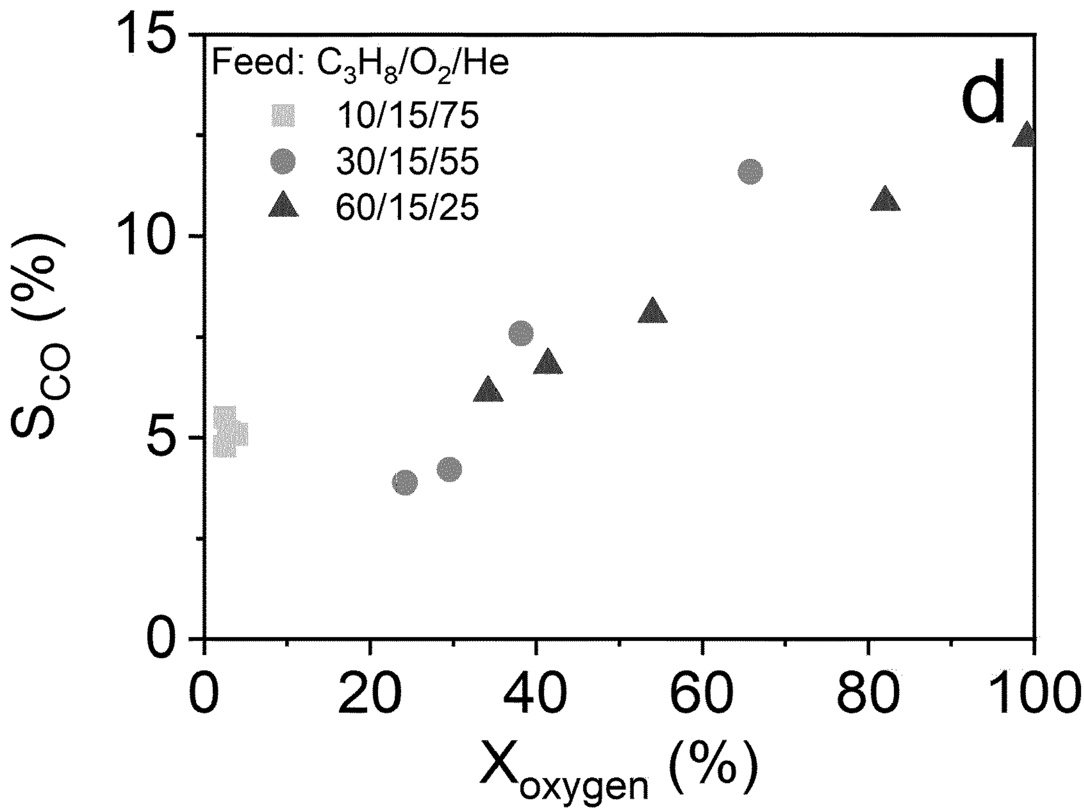
Feed: $C_3H_8/O_2/He$
10/15/75
30/15/55
60/15/25
d
$S_{CO}$ (%)
$X_{oxygen}$ (%)
0
5
10
15
0
20
40
60
80
100

**Figure 9.**

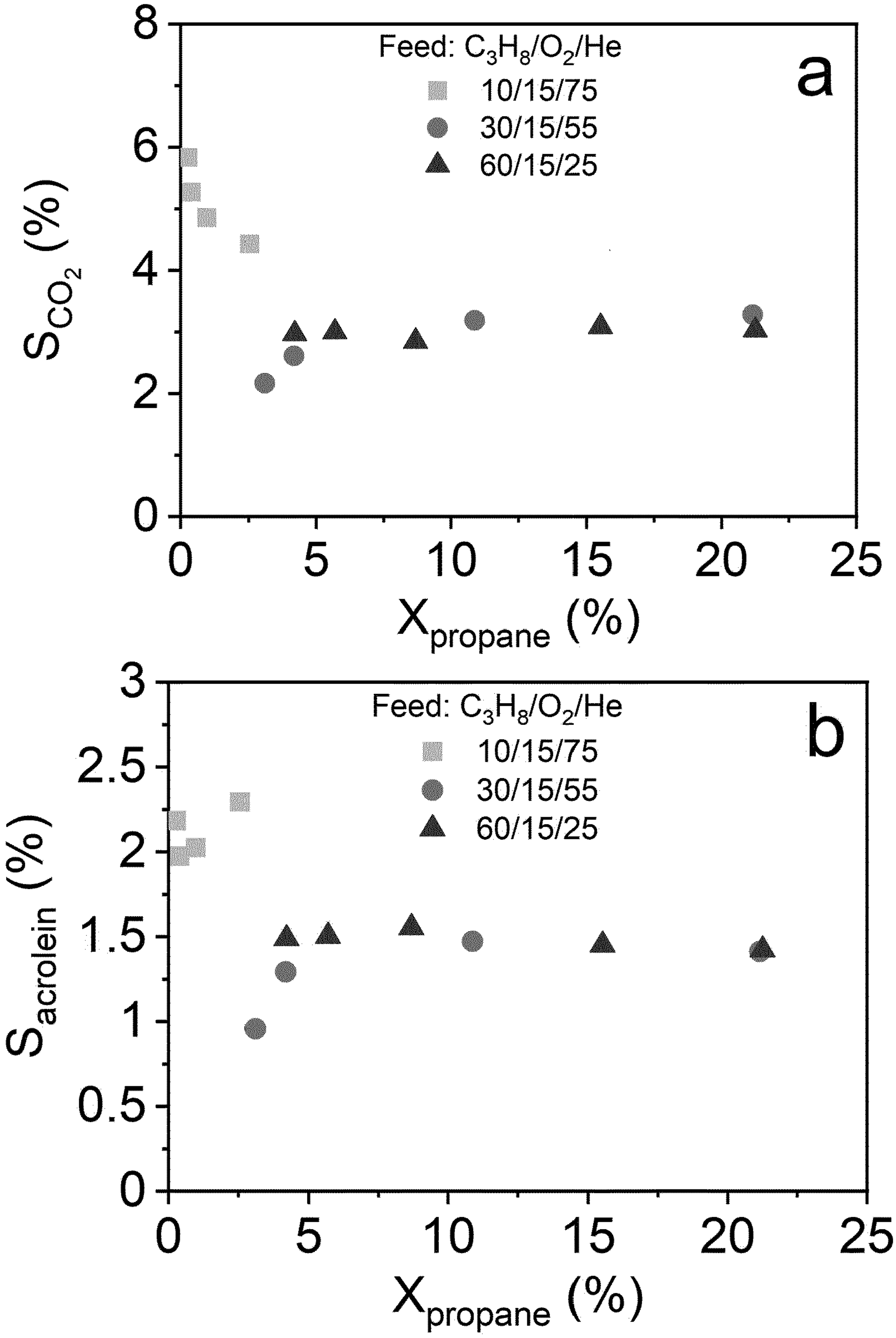
8
6
4
2
0
$S_{CO_2}$ (%)
Feed: $C_3H_8/O_2/He$
10/15/75
30/15/55
60/15/25
a
0
5
10
15
20
25
$X_{propane}$ (%)
3
2.5
2
1.5
1
0.5
0
$S_{acrolein}$ (%)
Feed: $C_3H_8/O_2/He$
10/15/75
30/15/55
60/15/25
b
0
5
10
15
20
25
$X_{propane}$ (%)

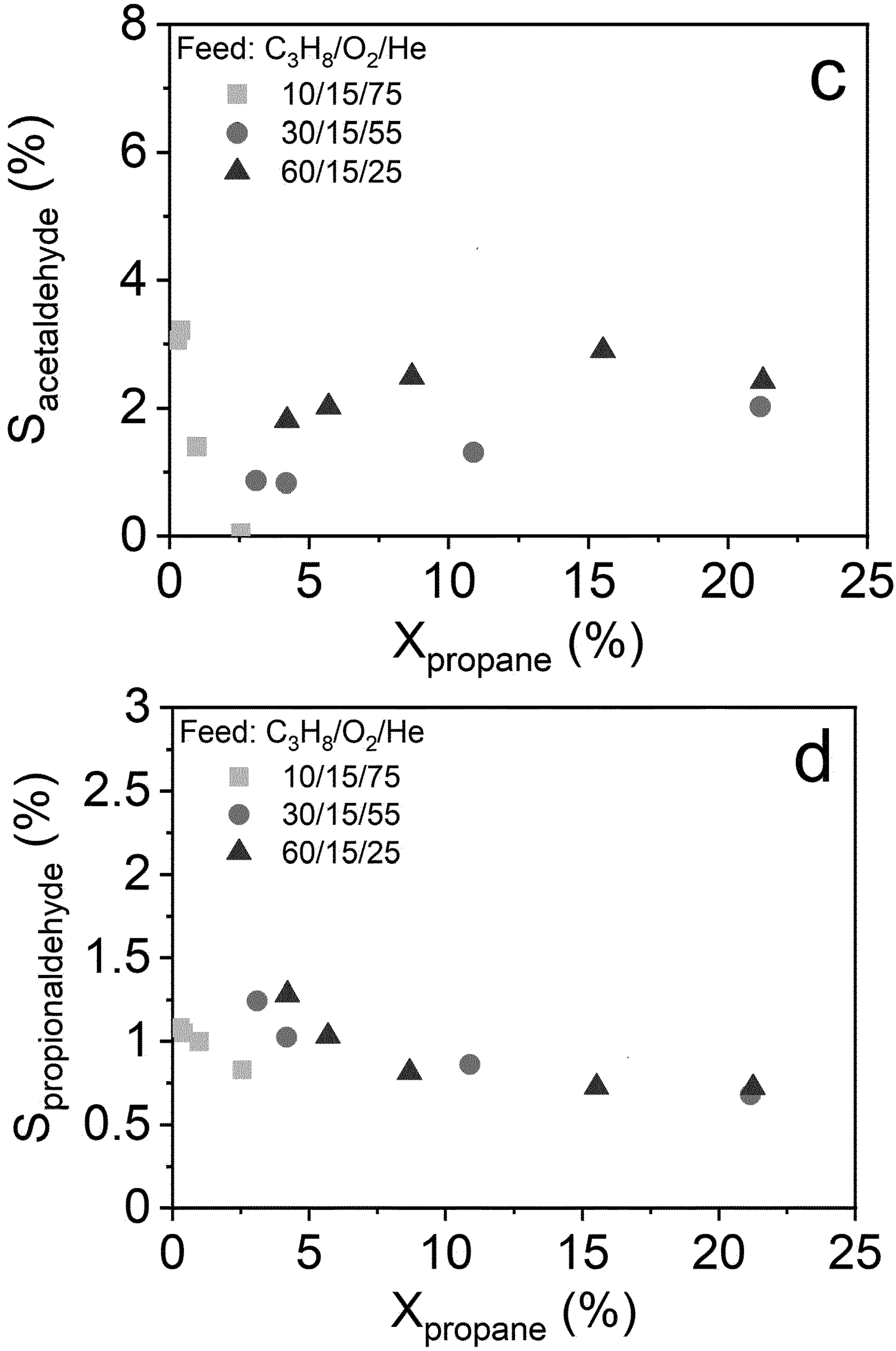
Feed: $C_3H_8/O_2/He$
10/15/75
30/15/55
60/15/25
c
$S_{acetaldehyde}$ (%)
$X_{propane}$ (%)
Feed: $C_3H_8/O_2/He$
10/15/75
30/15/55
60/15/25
d
$S_{propionaldehyde}$ (%)
$X_{propane}$ (%)

**Figure 10.**

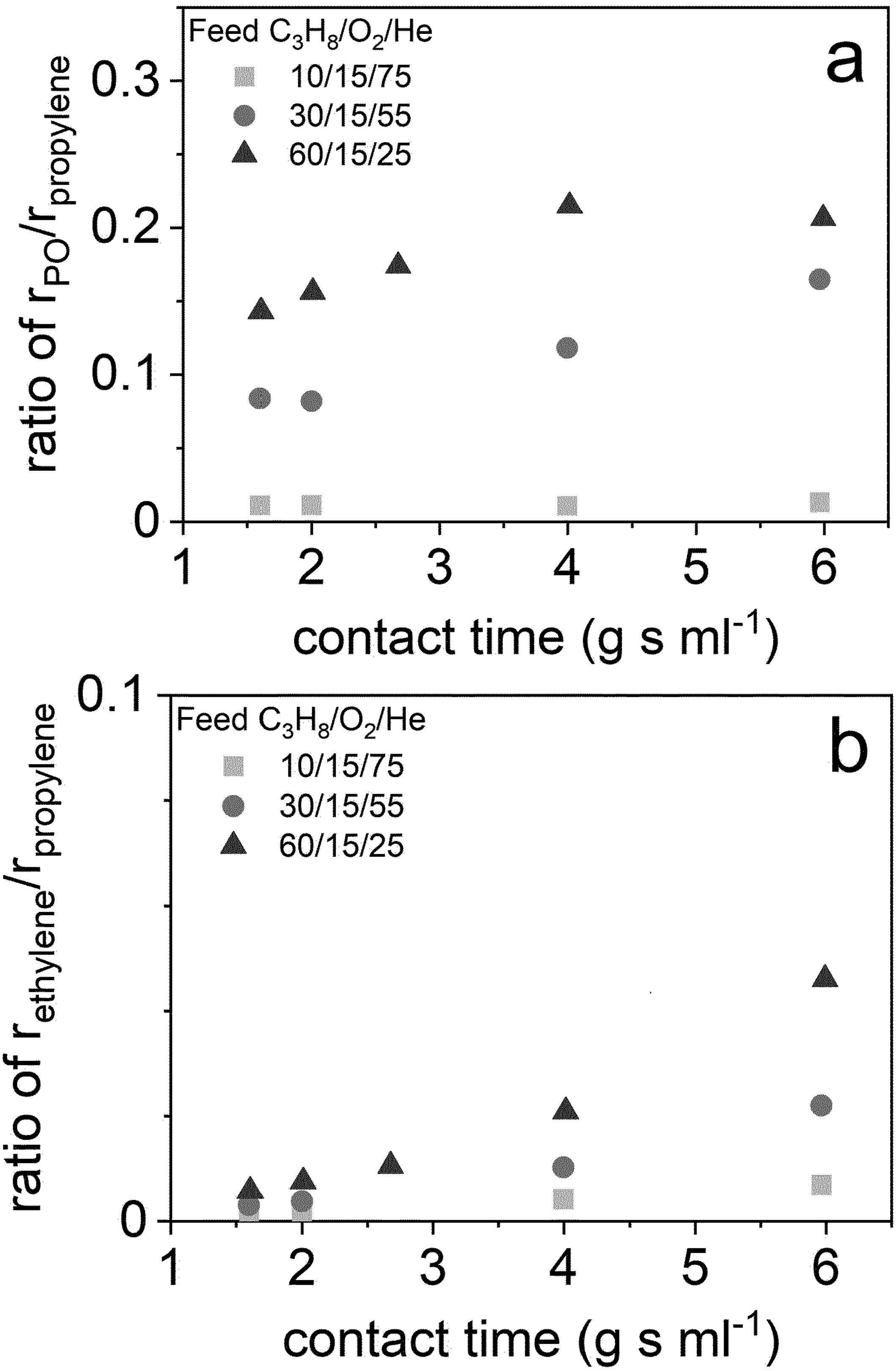
Feed $C_3H_8/O_2/He$
10/15/75
30/15/55
60/15/25
a
ratio of $r_{PO}/r_{propylene}$
0
0.1
0.2
0.3
1
2
3
4
5
6
contact time (g s $ml^{-1}$)
Feed $C_3H_8/O_2/He$
10/15/75
30/15/55
60/15/25
b
ratio of $r_{ethylene}/r_{propylene}$
0
0.1
1
2
3
4
5
6
contact time (g s $ml^{-1}$)

Figure 11

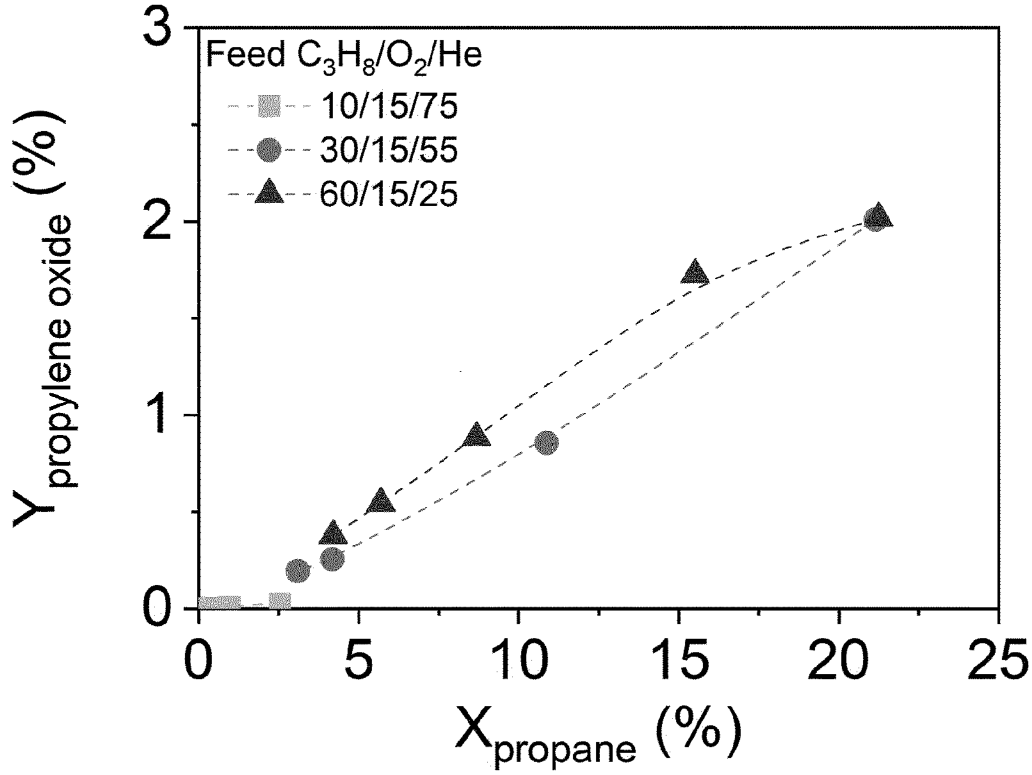


Figure 12.

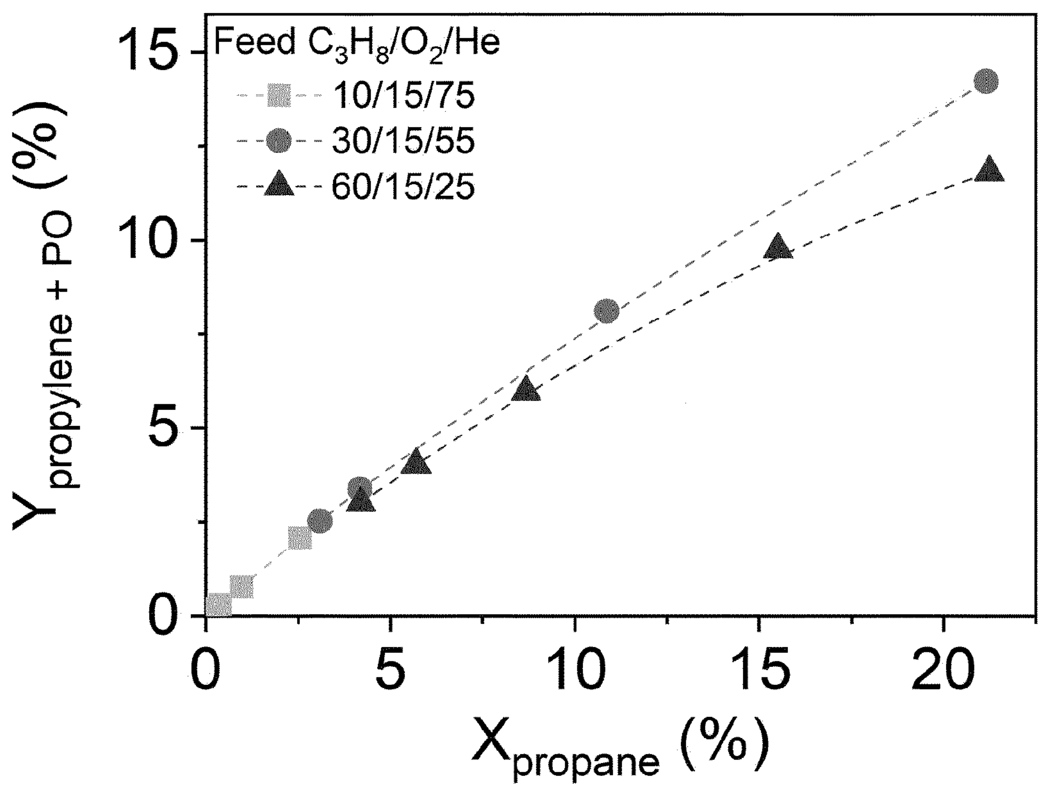

Figure 13.

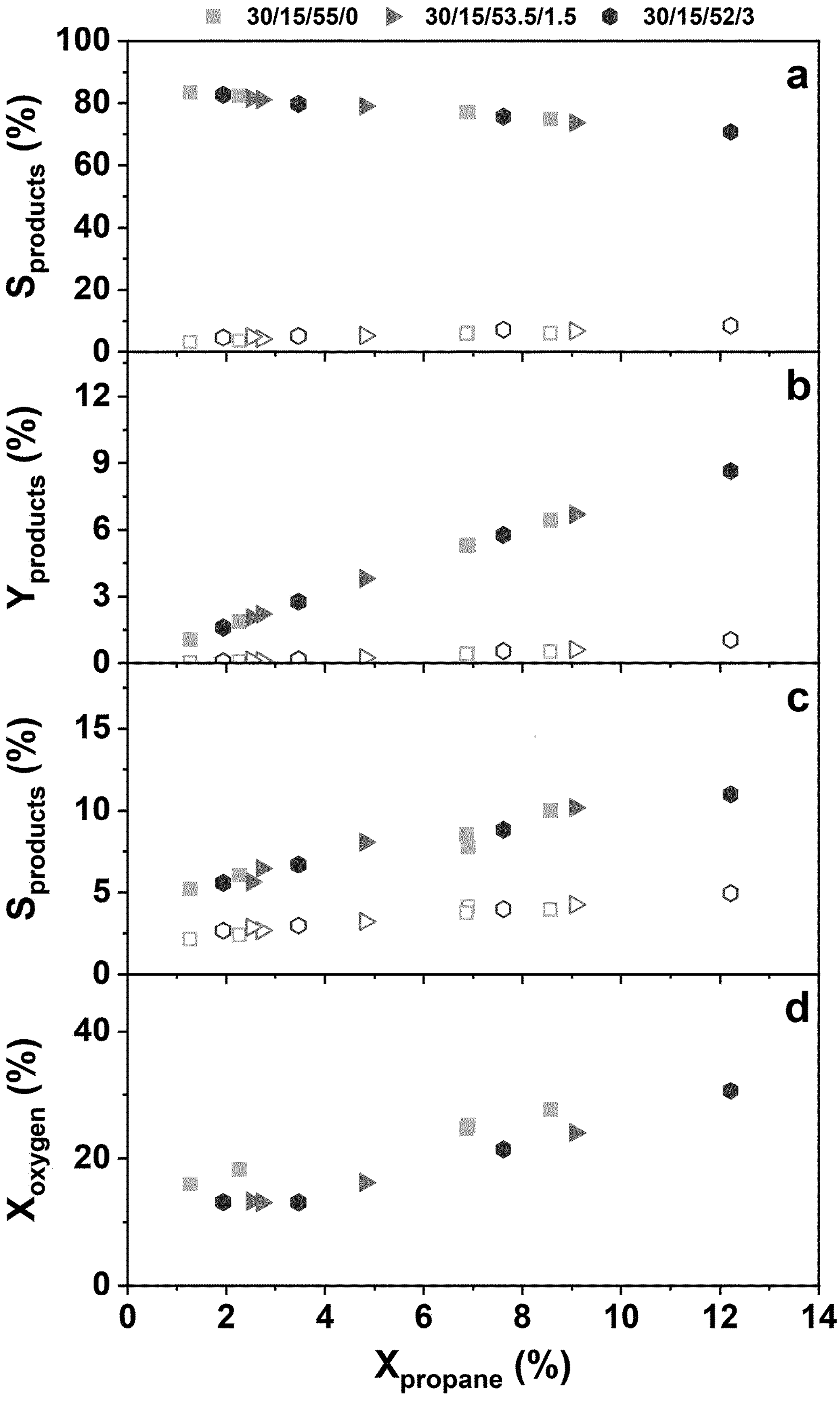
30/15/55/0
30/15/53.5/1.5
30/15/52/3
a
b
c
d
$S_{products}$ (%)
$Y_{products}$ (%)
$S_{products}$ (%)
$X_{oxygen}$ (%)
$X_{propane}$ (%)

Figure 14.

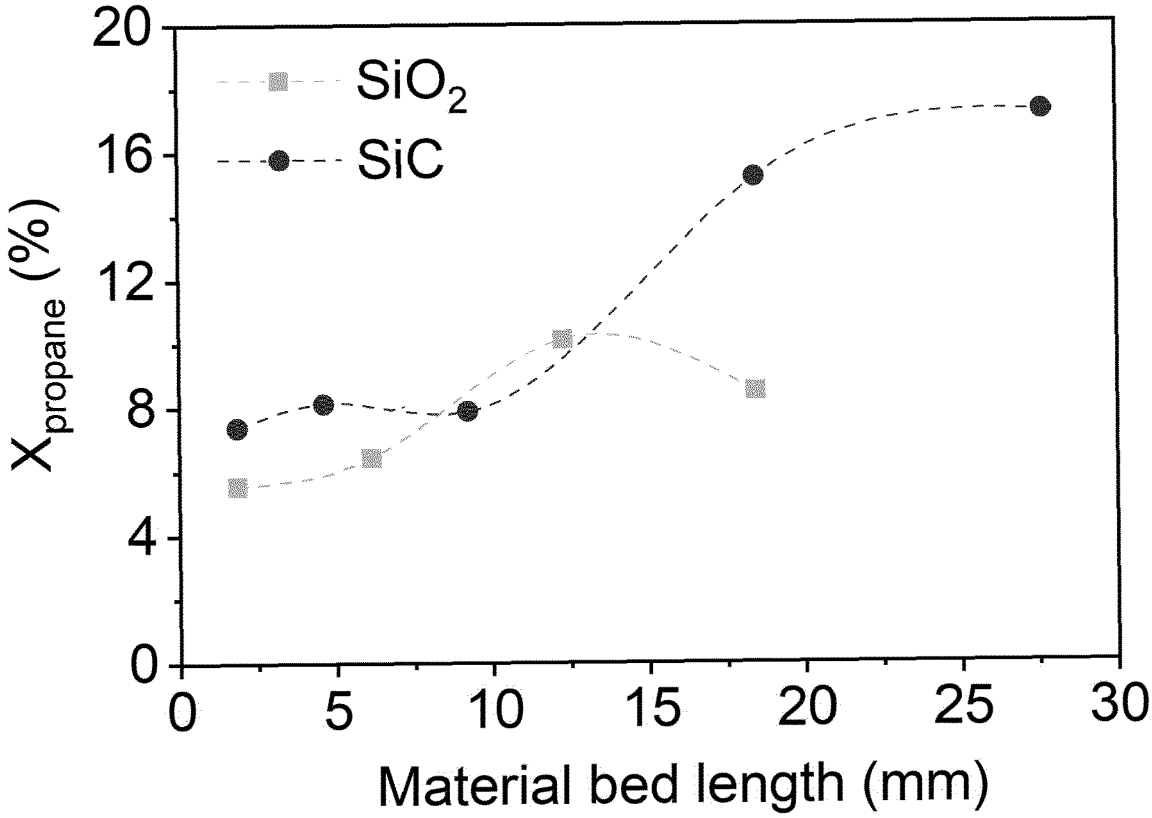
20
16
12
8
4
0
0
5
10
15
20
25
30
SiO2
SiC
Xpropane (%)
Material bed length (mm)

Figure 15.

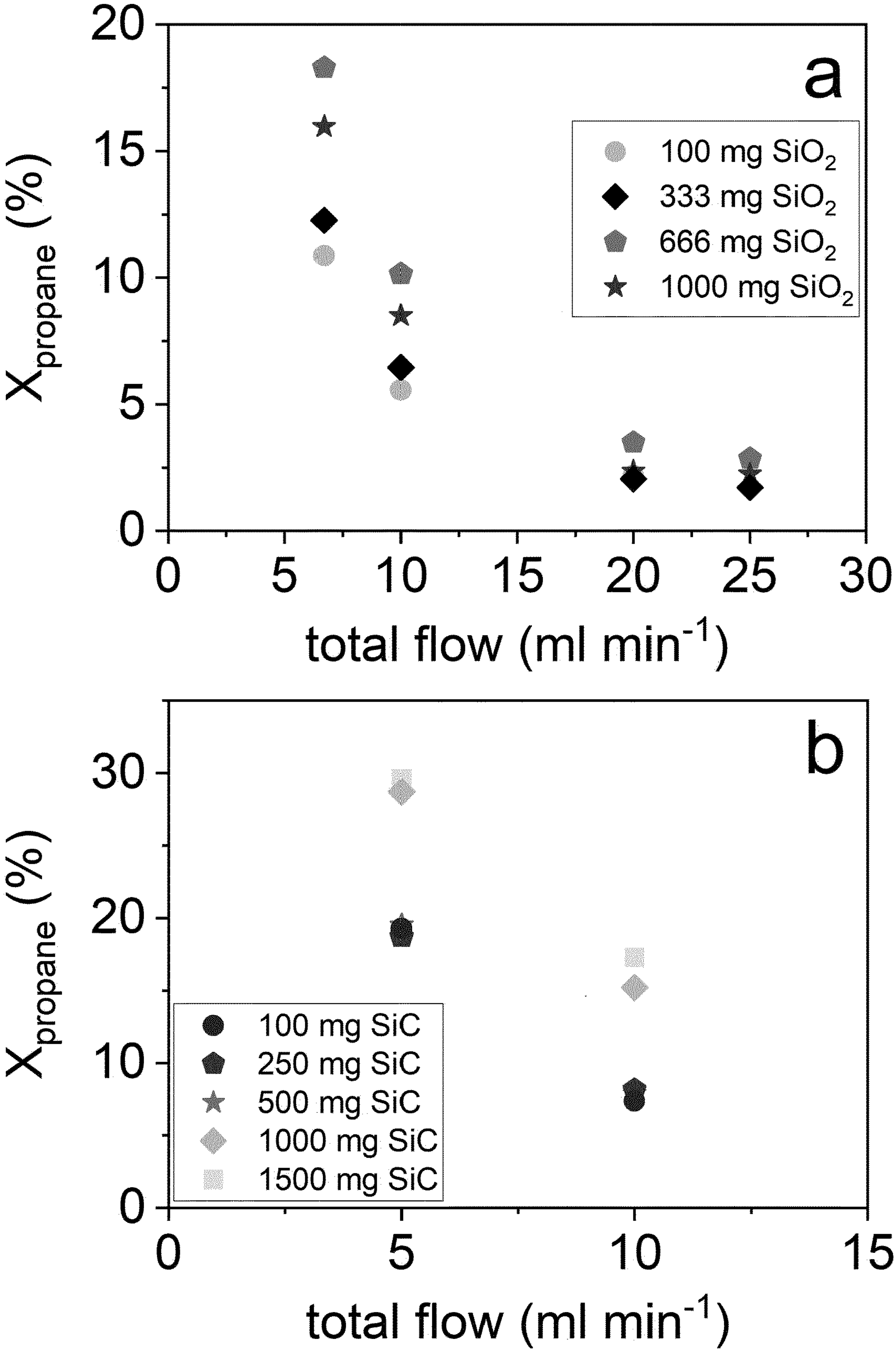
a
100 mg SiO2
333 mg SiO2
666 mg SiO2
1000 mg SiO2
Xpropane (%)
total flow (ml min-1)
b
100 mg SiC
250 mg SiC
500 mg SiC
1000 mg SiC
1500 mg SiC
Xpropane (%)
total flow (ml min-1)

Figure 16.

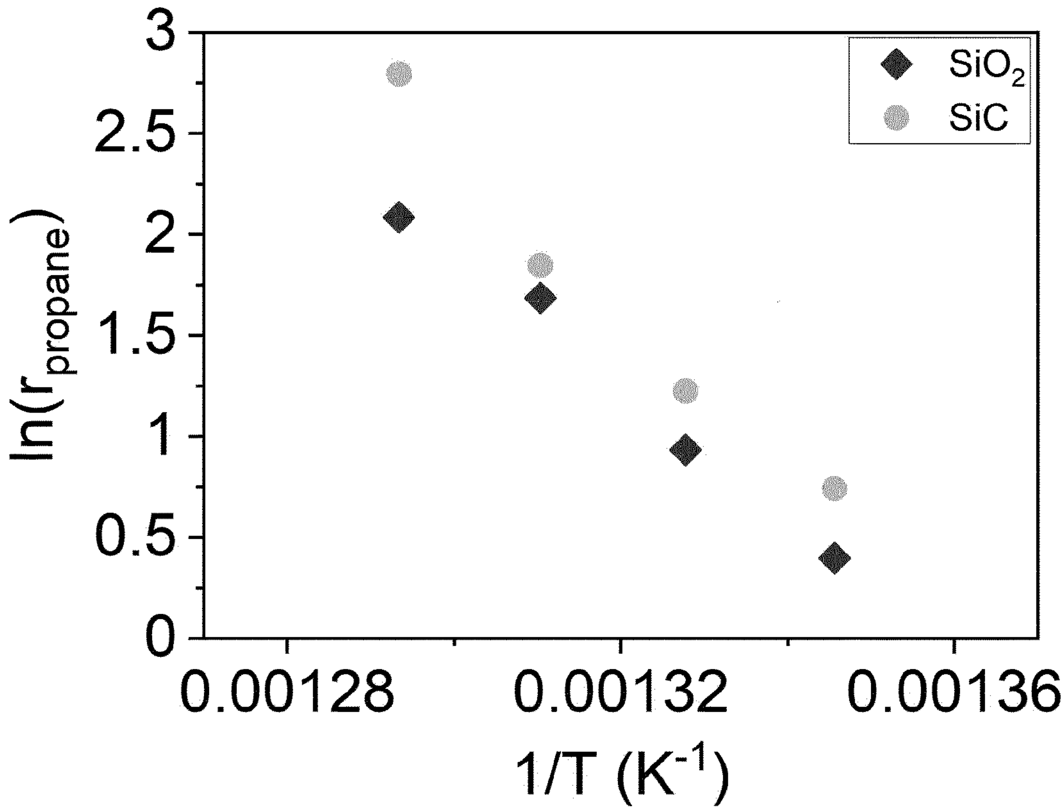


Figure 17.

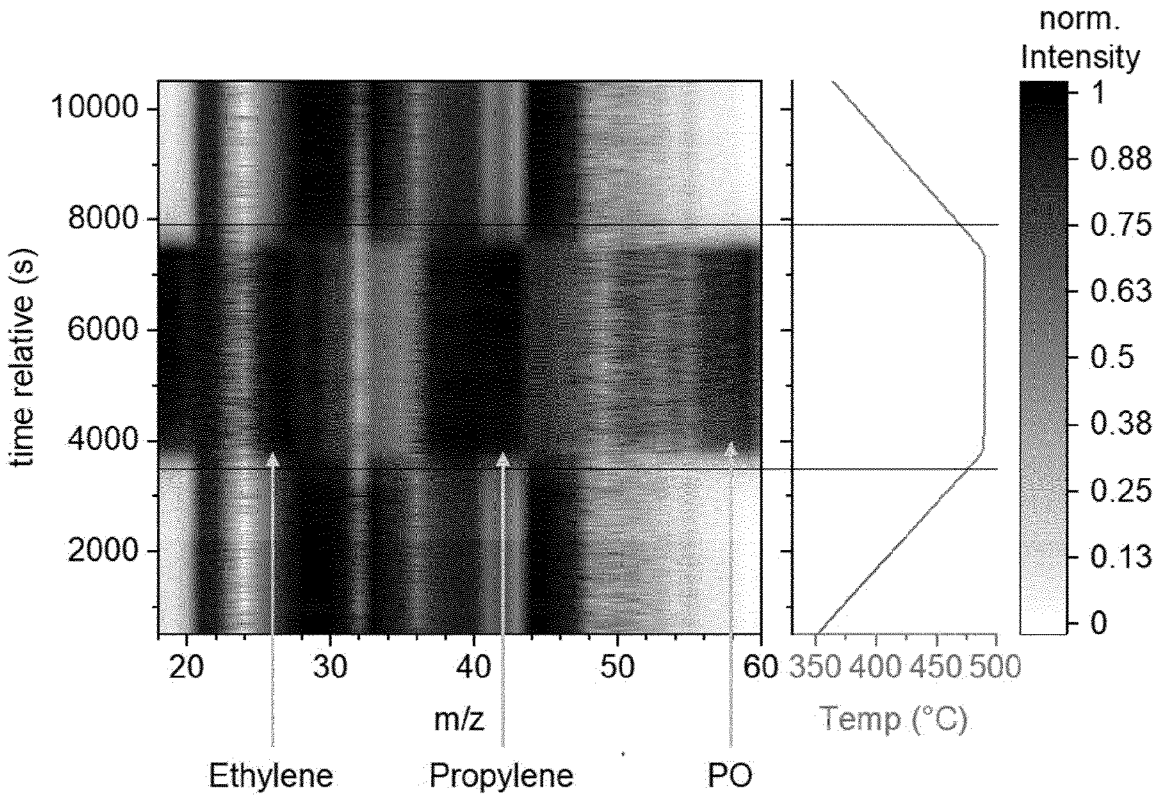

**Figure 18.**

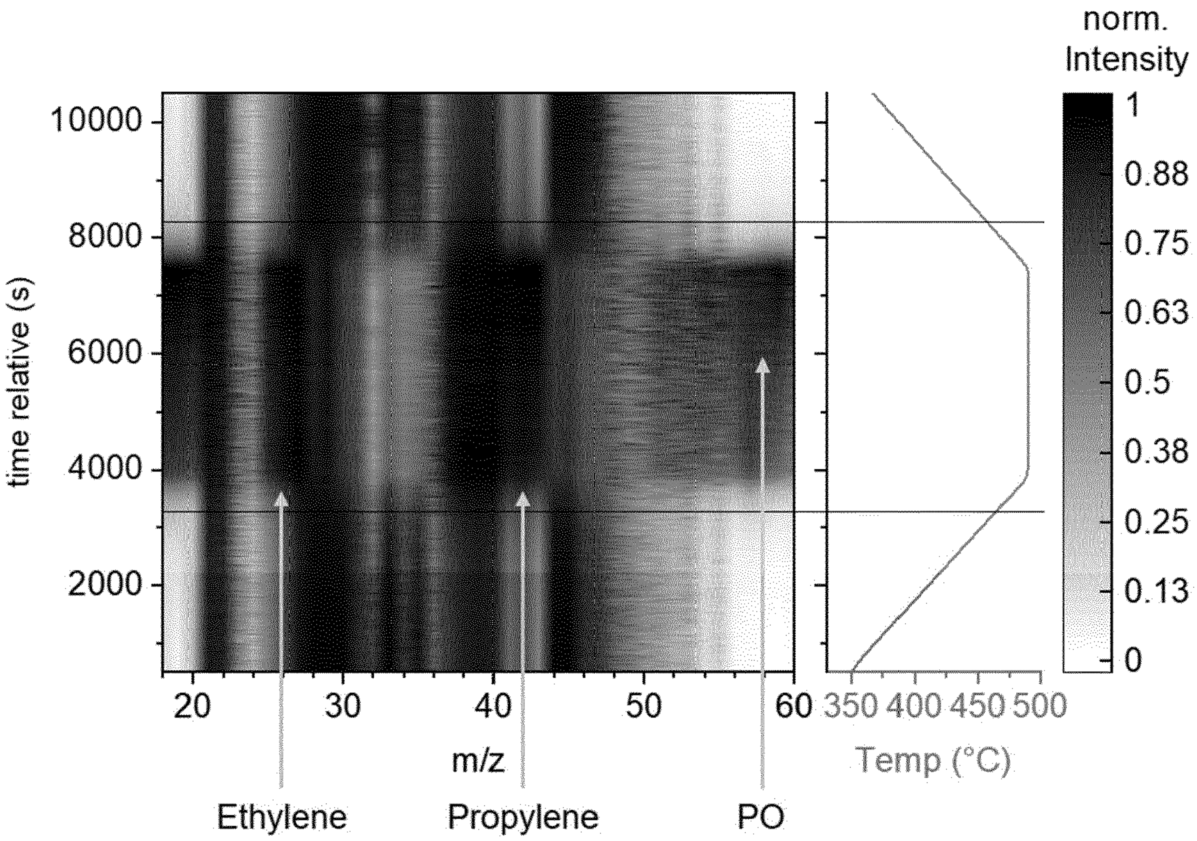
norm.
Intensity
1
0.88
0.75
0.63
0.5
0.38
0.25
0.13
0
10000
8000
6000
4000
2000
time relative (s)
20
30
40
50
60
m/z
350 400 450 500
Temp (°C)
Ethylene
Propylene
PO

**Figure 19.**

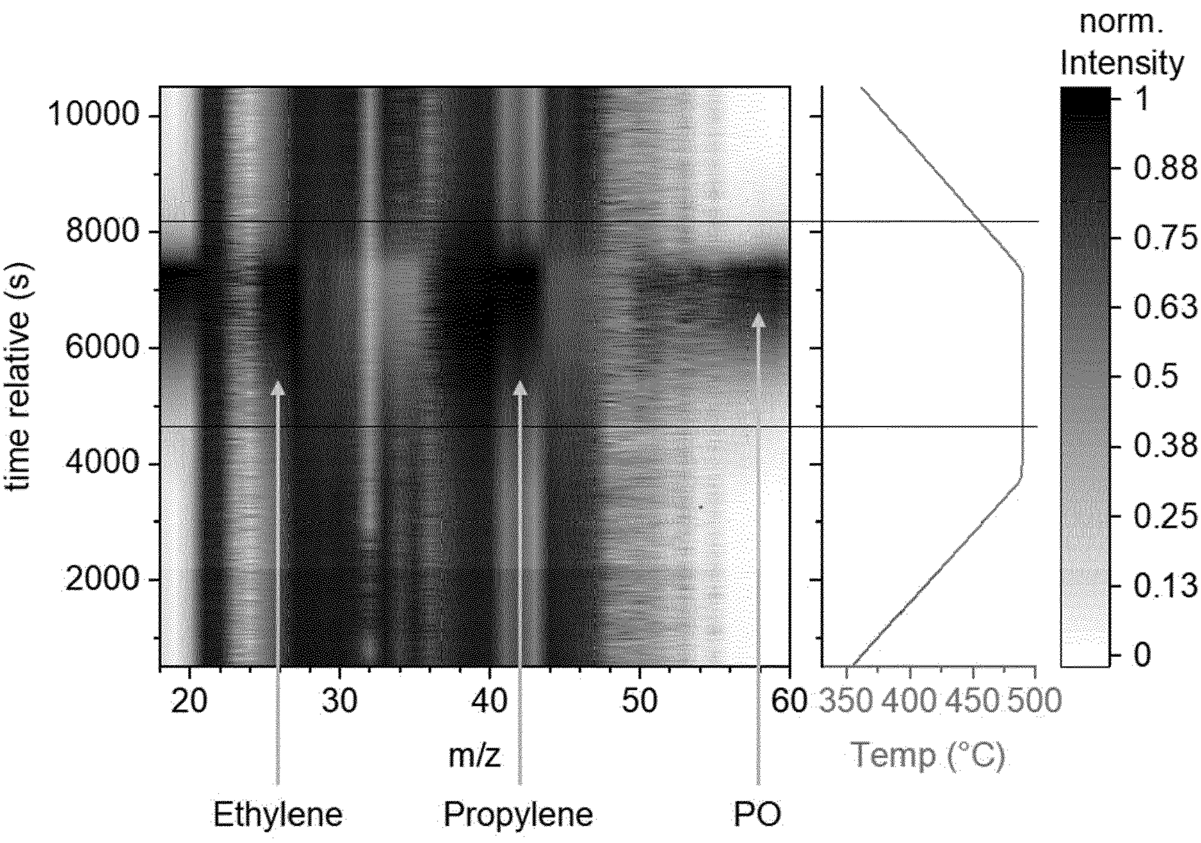
norm.
Intensity
1
0.88
0.75
0.63
0.5
0.38
0.25
0.13
0
10000
8000
6000
4000
2000
time relative (s)
20
30
40
50
60
m/z
350
400
450
500
Temp (°C)
Ethylene
Propylene
PO

Figure 20.

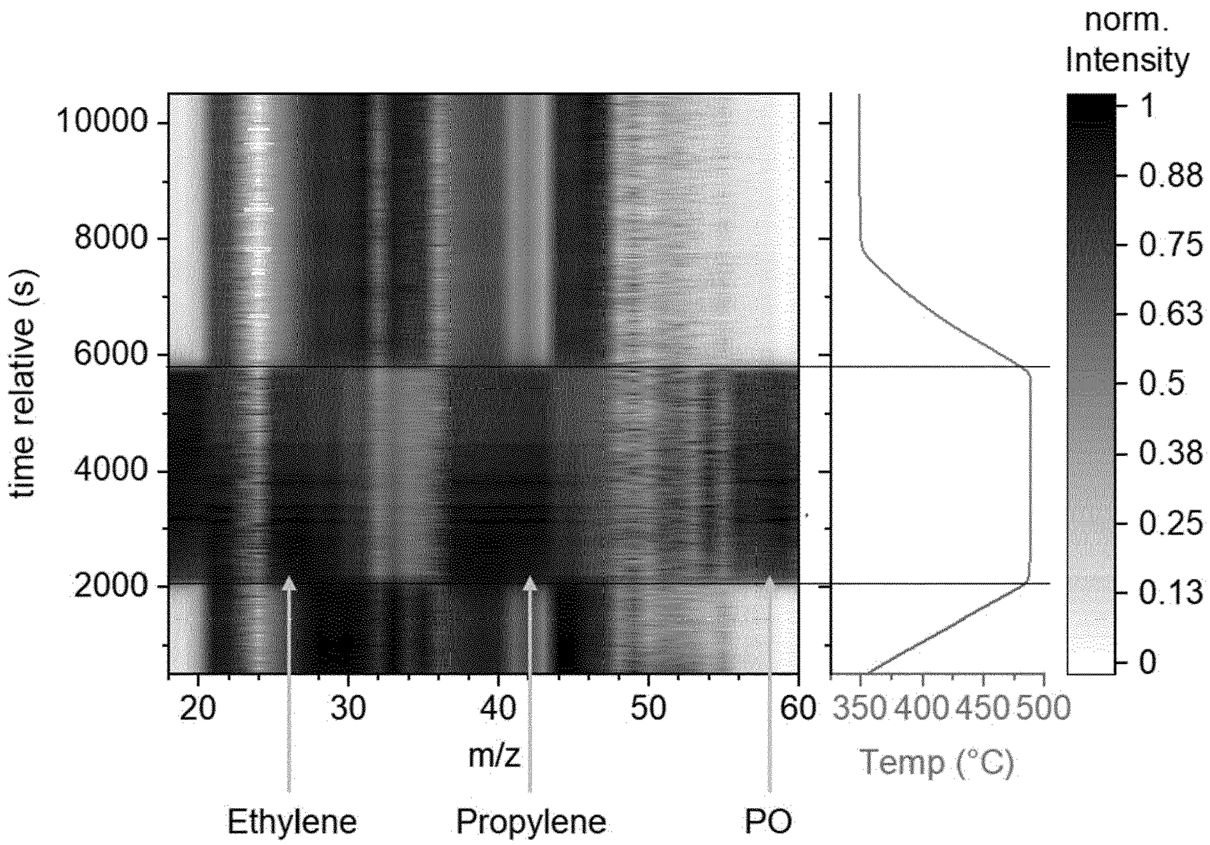
norm.
Intensity
1
0.88
0.75
0.63
0.5
0.38
0.25
0.13
0
10000
8000
6000
4000
2000
time relative (s)
20
30
40
50
60
m/z
350
400
450
500
Temp (°C)
Ethylene
Propylene
PO

**Figure 21.**

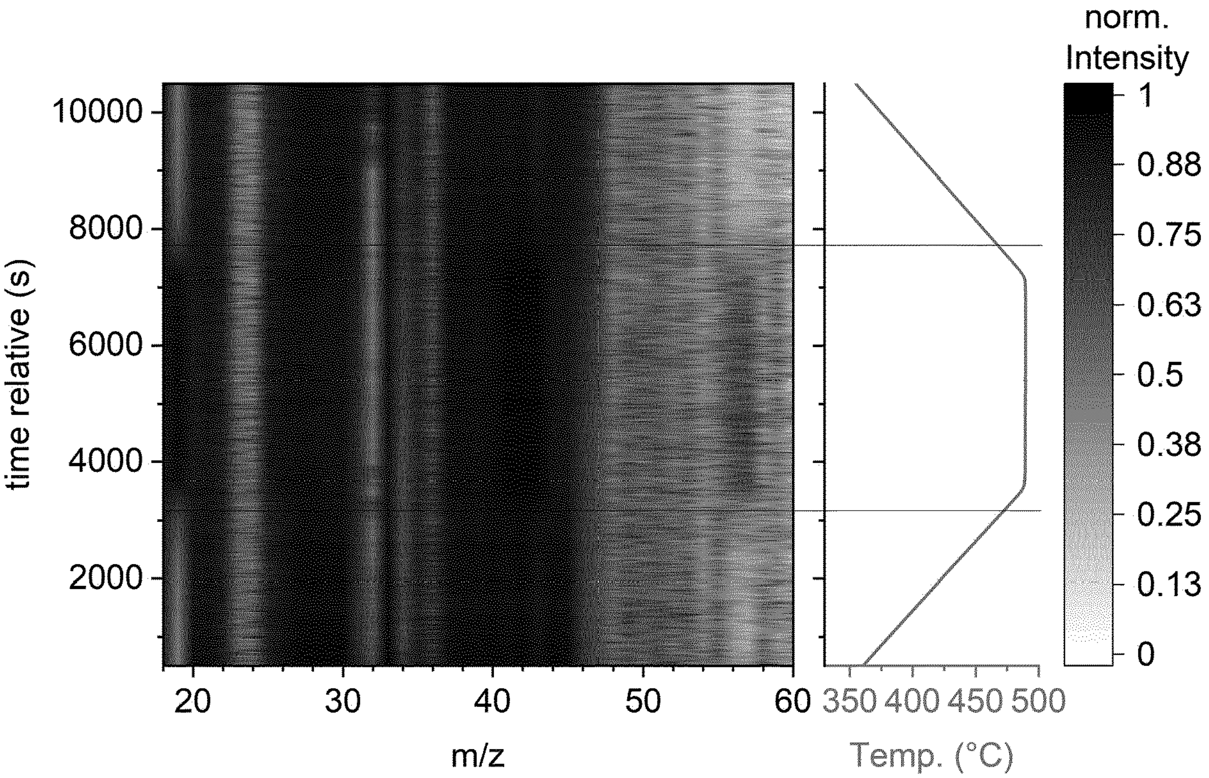
norm.
Intensity
1
0.88
0.75
0.63
0.5
0.38
0.25
0.13
0
10000
8000
6000
4000
2000
time relative (s)
20
30
40
50
60
m/z
350
400
450
500
Temp. (°C)

**Figure 22.**

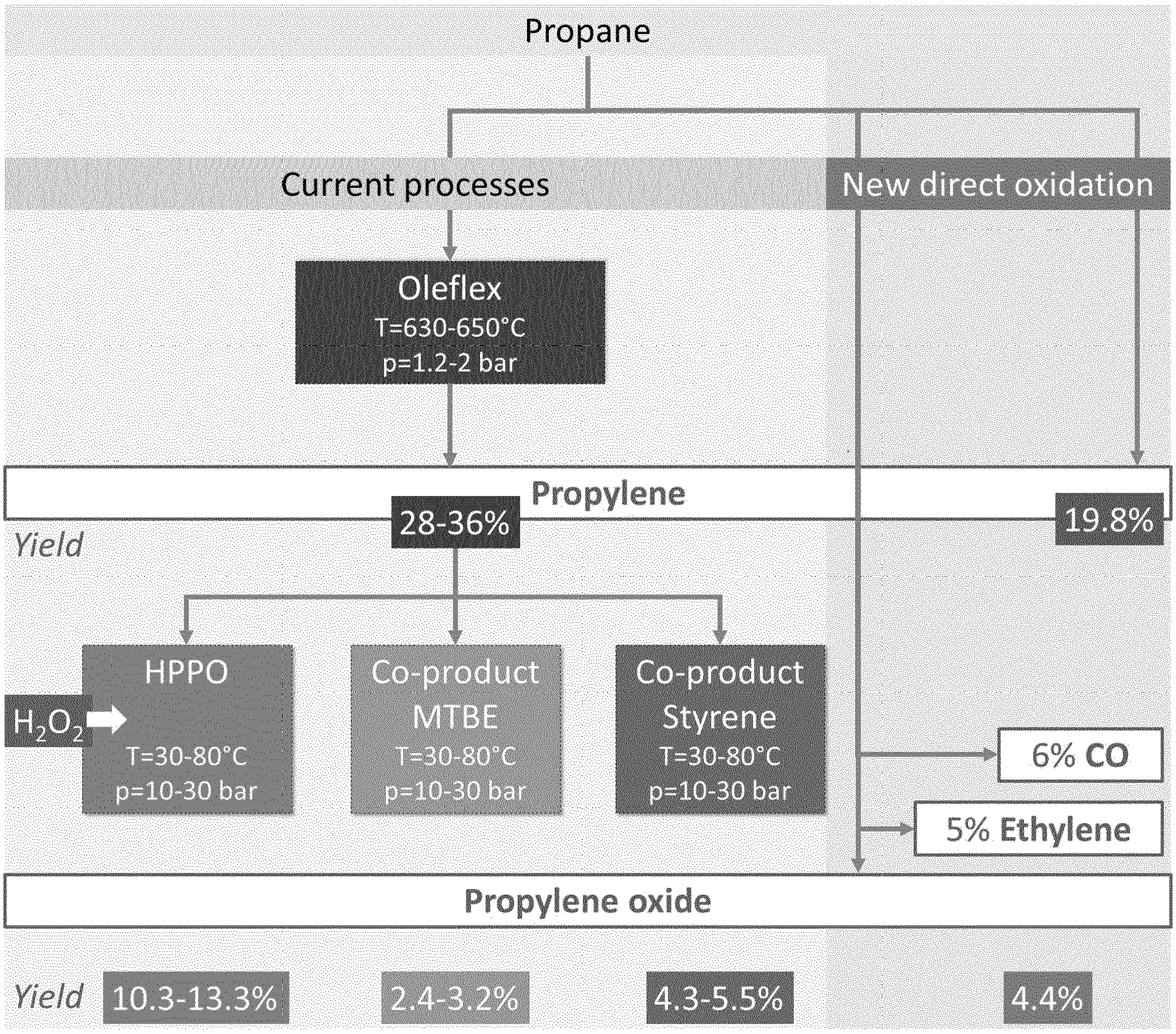
Propane
Current processes
New direct oxidation
Oleflex
T=630-650°C
p=1.2-2 bar
Propylene
Yield
28-36%
19.8%
HPPO
$H_2O_2$
T=30-80°C
p=10-30 bar
Co-product
MTBE
T=30-80°C
p=10-30 bar
Co-product
Styrene
T=30-80°C
p=10-30 bar
6% CO
5% Ethylene
Propylene oxide
Yield
10.3-13.3%
2.4-3.2%
4.3-5.5%
4.4%

**Figure 23.**

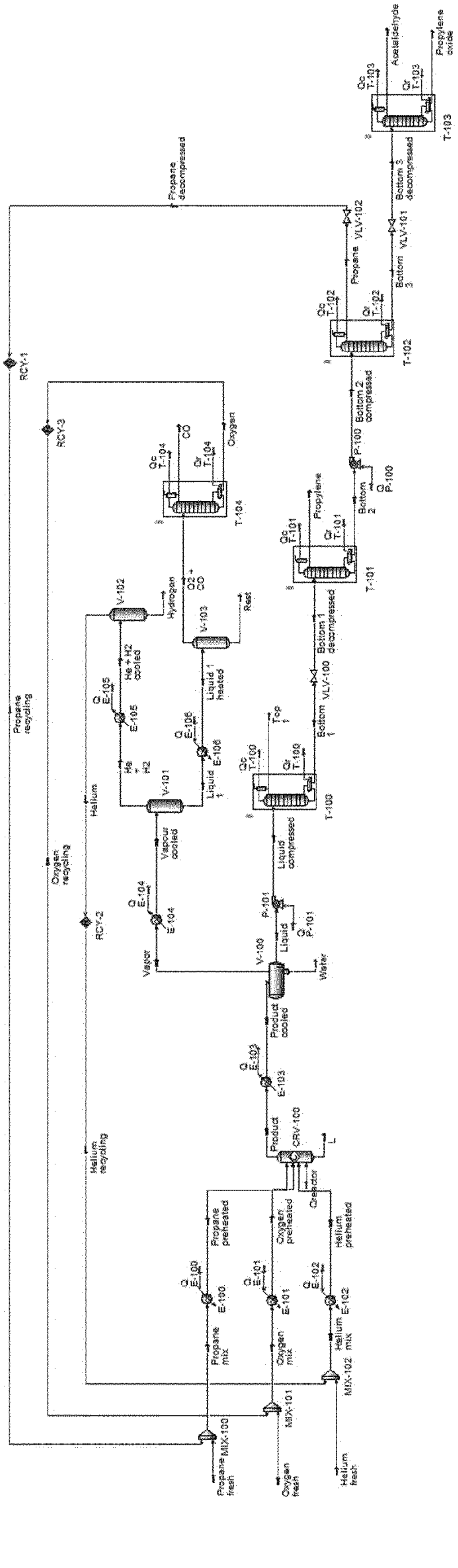
Propane fresh
MIX-100
Oxygen fresh
MIX-101
Helium fresh
MIX-102
Propane mix
Oxygen mix
Helium mix
E-100
E-101
E-102
Propane preheated
Oxygen preheated
Helium preheated
Qreactor
CRV-100
Product
E-103
Product cooled
V-100
Water
Vapor
Liquid
P-101
Liquid compressed
T-100
Top 1
Bottom 1
VLV-100
Bottom 1 decompressed
T-101
Propylene
Bottom 2
P-100
Bottom 2 compressed
T-102
Propane
VLV-102
Propane decompressed
Bottom 3
VLV-101
Bottom 3 decompressed
T-103
Acetaldehyde
Propylene oxide
E-104
Vapour cooled
V-101
He + H2
E-105
He + H2 cooled
V-102
Hydrogen
Liquid 1
E-106
Liquid 1 heated
V-103
Rest
O2 + CO
T-104
CO
Oxygen
Helium
RCY-1
RCY-2
RCY-3
Propane recycling
Oxygen recycling
Helium recycling

Europäisches Patentamt
European Patent Office
Office européen des brevets

# EUROPEAN SEARCH REPORT

Application Number
EP 21 20 7457

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | A. HELD ET AL.: "Epoxidation of propane with oxygen and/or nitrous oxide over silica supported vanadum oxide.", JOURNAL OF CATALYSIS, vol. 404, 27 September 2021 (2021-09-27), pages 231-243, XP086901185, DOI: 10.1016/j.jcat.2021.09.022 | 1,2,5,6, 10,13-15 | INV. C07D301/22 |
| A | * paragraph [3.2.2]; table 4 * ----- | 9,11 | |
| X | US 2002/161249 A1 (MUL ET. AL.) 31 October 2002 (2002-10-31) | 1,3-8, 10,13-15 | |
| A | * claims; example 4 * ----- | 9,11 | |
| X | US 4 885 374 A (PENNINGTON) 5 December 1989 (1989-12-05) * column 3, line 10 - line 18; claims; example 2 * ----- | 1,5,7,8, 12,13 | |
| X | CN 101 020 670 A (UVIVERSITY OF DALIAN MARITIME) 22 August 2007 (2007-08-22) * claims; examples * ----- | 1,6-8,12 | TECHNICAL FIELDS SEARCHED (IPC) |
| X | CN 1 446 626 A (UNIVERSITY OF HUADONG TECHNOLOGY) 8 October 2003 (2003-10-08) * claims; examples * ----- | 1,3-5, 10,13 | C07D |

The present search report has been drawn up for all claims

1

| Place of search | Date of completion of the search | Examiner |
|---|---|---|
| Munich | 7 April 2022 | Helps, Ian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO.

EP 21 20 7457

This annex lists the patent family members relating to the patent documents cited in the above-mentioned European search report. The members are as contained in the European Patent Office EDP file on
The European Patent Office is in no way liable for these particulars which are merely given for the purpose of information.

07-04-2022

| Patent document cited in search report | Publication date | Patent family member(s) | Publication date |
|---|---|---|---|
| US 2002161249 A1 | 31-10-2002 | US 2002161249 A1 | 31-10-2002 |
| | | US 2003130552 A1 | 10-07-2003 |
| | | WO 02068400 A2 | 06-09-2002 |
| US 4885374 A | 05-12-1989 | NONE | |
| CN 101020670 A | 22-08-2007 | NONE | |
| CN 1446626 A | 08-10-2003 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *Proc. Natl. Acad. Sci. U.S.A.,* 2019, vol. 116, 11187-11194
- *J. Energy Chem.,* 2021, vol. 56, 193-202
- **BAER et al.** Propylene Oxide. Wiley Online Library, 2012
- *Nature,* 2020, vol. 586, 708-713
- *Catal. Rev.,* 2015, vol. 57, 306-344
- *J. Catal.,* 2016, vol. 336, 23-32
- *ChemCatChem,* 2017, vol. 9, 3623-3626
- *ACS Catal,* 2014, vol. 4, 3357-3380
- *Science,* 2016, vol. 354, 1570-1573
- *Ind. Eng. Chem. Res.,* 2019, vol. 58, 2170-2180
- *ChemCatChem,* 2017, vol. 9, 1788-1793
- *Chem. Eng. Sci.,* 2018, vol. 186, 142-151
- *Chem. Commun.,* 2020, vol. 56, 9882-9885